# EUROPEAN PATENT APPLICATION

(11) **EP 3 275 462 A1**
(43) Date of publication of application: **31.01.2018**
(21) Application number: 16382364.4
(22) Date of filing: 27.07.2016
(51) Int. Cl.: A61K 39/21, C07K 14/16, A61K 39/12, A61K 39/00

(54) **VIRUS-LIKE PARTICLES WITH HIGH-DENSITY COATING FOR THE PRODUCTION OF NEUTRALIZING ANTIBODIES**

(71) Applicant: Fundació Privada Institut de Recerca de la SIDA-Caixa, 08916 Badalona (ES)
(72) Inventor: CARRILLO MOLINA, Jorge, 08912 Badalona (ES); MOLINOS-ALBERT, Luis M., 03003 Alicante (ES); BLANCO ARBUÉS, Julián Miguel, 08500 Vic (ES)
(74) Representative: ABG Patentes, S.L.

(57) **Abstract**

The invention relates to a fusion protein comprising a HIV gag polypeptide or a functionally equivalent variant thereof and an immunogenic polypeptide, wherein the immunogenic polypeptide is located N-terminal to the HIV gag polypeptide or to the functionally equivalent variant thereof, to a polynucleotide, a vector, a host cell and an immunogenic composition or vaccine composition and the their use in medicine, particularly in in the prevention and/or treatment of a disease caused by an infection in a subject, wherein the infection is by a pathogen which contains the immunogenic polypeptide forming part of the fusion protein or a region thereof.

SEQUENCE LISTING

<110> LABORATORIOS DEL DR. ESTEVE, S.A.
FUNDACIÓ PRIVADA INSTITUT DE RECERCA DE LA SIDA \226 CAIXA

<120> VIRUS-LIKE PARTICLES WITH HIGH-DENSITY COATING FOR THE PRODUCTION
OF NEUTRALIZING ANTIBODIES

<130> P13141EP00

<160> 29

<170> PatentIn version 3.5

<210> 1
<211> 500
<212> PRT
<213> Human immunodeficiency virus type 1(HXB2)

<400> 1

Met Gly Ala Arg Ala Ser Val Leu Ser Gly Gly Glu Leu Asp Arg Trp
1               5                   10                  15

Glu Lys Ile Arg Leu Arg Pro Gly Gly Lys Lys Lys Tyr Lys Leu Lys
            20                  25                  30

His Ile Val Trp Ala Ser Arg Glu Leu Glu Arg Phe Ala Val Asn Pro
        35                  40                  45

Gly Leu Leu Glu Thr Ser Glu Gly Cys Arg Gln Ile Leu Gly Gln Leu
    50                  55                  60

Gln Pro Ser Leu Gln Thr Gly Ser Glu Glu Leu Arg Ser Leu Tyr Asn
65                  70                  75                  80

Thr Val Ala Thr Leu Tyr Cys Val His Gln Arg Ile Glu Ile Lys Asp
                85                  90                  95

Thr Lys Glu Ala Leu Asp Lys Ile Glu Glu Glu Gln Asn Lys Ser Lys
            100                 105                 110

Lys Lys Ala Gln Gln Ala Ala Ala Asp Thr Gly His Ser Asn Gln Val
        115                 120                 125

Ser Gln Asn Tyr Pro Ile Val Gln Asn Ile Gln Gly Gln Met Val His
    130                 135                 140

Gln Ala Ile Ser Pro Arg Thr Leu Asn Ala Trp Val Lys Val Val Glu
145                 150                 155                 160

Glu Lys Ala Phe Ser Pro Glu Val Ile Pro Met Phe Ser Ala Leu Ser
                165                 170                 175

Glu Gly Ala Thr Pro Gln Asp Leu Asn Thr Met Leu Asn Thr Val Gly
        180                 185                 190

Gly His Gln Ala Ala Met Gln Met Leu Lys Glu Thr Ile Asn Glu Glu
    195                 200                 205

Ala Ala Glu Trp Asp Arg Val His Pro Val His Ala Gly Pro Ile Ala
    210                 215                 220

Pro Gly Gln Met Arg Glu Pro Arg Gly Ser Asp Ile Ala Gly Thr Thr
225                 230                 235                 240

Ser Thr Leu Gln Glu Gln Ile Gly Trp Met Thr Asn Asn Pro Pro Ile
                245                 250                 255

Pro Val Gly Glu Ile Tyr Lys Arg Trp Ile Ile Leu Gly Leu Asn Lys
        260                 265                 270

Ile Val Arg Met Tyr Ser Pro Thr Ser Ile Leu Asp Ile Arg Gln Gly
    275                 280                 285

Pro Lys Glu Pro Phe Arg Asp Tyr Val Asp Arg Phe Tyr Lys Thr Leu
    290                 295                 300

Arg Ala Glu Gln Ala Ser Gln Glu Val Lys Asn Trp Met Thr Glu Thr
305                 310                 315                 320

Leu Leu Val Gln Asn Ala Asn Pro Asp Cys Lys Thr Ile Leu Lys Ala
                325                 330                 335

Leu Gly Pro Ala Ala Thr Leu Glu Glu Met Met Thr Ala Cys Gln Gly
        340                 345                 350

Val Gly Gly Pro Gly His Lys Ala Arg Val Leu Ala Glu Ala Met Ser
    355                 360                 365

Gln Val Thr Asn Ser Ala Thr Ile Met Met Gln Arg Gly Asn Phe Arg
    370                 375                 380

Asn Gln Arg Lys Ile Val Lys Cys Phe Asn Cys Gly Lys Glu Gly His
385                 390                 395                 400

Thr Ala Arg Asn Cys Arg Ala Pro Arg Lys Lys Gly Cys Trp Lys Cys
                405                 410                 415

Gly Lys Glu Gly His Gln Met Lys Asp Cys Thr Glu Arg Gln Ala Asn

420                 425                 430

Phe Leu Gly Lys Ile Trp Pro Ser Tyr Lys Gly Arg Pro Gly Asn Phe
        435                 440                 445

Leu Gln Ser Arg Pro Glu Pro Thr Ala Pro Pro Glu Glu Ser Phe Arg
    450                 455                 460

Ser Gly Val Glu Thr Thr Thr Pro Pro Gln Lys Gln Glu Pro Ile Asp
465                 470                 475                 480

Lys Glu Leu Tyr Pro Leu Thr Ser Leu Arg Ser Leu Phe Gly Asn Asp
                485                 490                 495

Pro Ser Ser Gln
            500

<210>  2
<211>  499
<212>  PRT
<213>  Artificial Sequence

<220>
<223>  Gag polypeptide lacking the myristoylation sequence and the first
       methionine

<400>  2

Gly Ala Arg Ala Ser Val Leu Ser Gly Gly Glu Leu Asp Arg Trp Gly
1               5                   10                  15

Lys Ile Arg Leu Arg Pro Gly Gly Lys Lys Lys Tyr Lys Leu Lys His
            20                  25                  30

Ile Val Trp Ala Ser Arg Glu Leu Glu Arg Phe Ala Val Asn Pro Gly
        35                  40                  45

Leu Leu Glu Thr Ser Glu Gly Cys Arg Gln Ile Leu Gly Gln Leu Gln
    50                  55                  60

Pro Ser Leu Gln Thr Gly Ser Glu Glu Leu Arg Ser Leu Tyr Asn Thr
65                  70                  75                  80

Val Ala Thr Leu Tyr Cys Val His Gln Arg Ile Glu Ile Lys Asp Thr
                85                  90                  95

Lys Glu Ala Leu Asp Lys Ile Glu Glu Glu Gln Asn Lys Ser Lys Lys
            100                 105                 110

Lys Ala Gln Gln Ala Ala Ala Asp Thr Gly His Ser Asn Gln Val Ser

115                 120                 125

Gln Asn Tyr Pro Ile Val Gln Asn Ile Gln Gly Gln Met Val His Gln
    130                 135                 140

Ala Ile Ser Pro Arg Thr Leu Asn Ala Trp Val Lys Val Val Glu Glu
145                 150                 155                 160

Lys Ala Phe Ser Pro Glu Val Ile Pro Met Phe Ser Ala Leu Ser Glu
                165                 170                 175

Gly Ala Thr Pro Gln Asp Leu Asn Thr Met Leu Asn Thr Val Gly Gly
            180                 185                 190

His Gln Ala Ala Met Gln Met Leu Lys Glu Thr Ile Asn Glu Glu Ala
        195                 200                 205

Ala Glu Trp Asp Arg Val His Pro Val His Ala Gly Pro Ile Ala Pro
    210                 215                 220

Gly Gln Met Arg Glu Pro Arg Gly Ser Asp Ile Ala Gly Thr Thr Ser
225                 230                 235                 240

Thr Leu Gln Glu Gln Ile Gly Trp Met Thr Asn Asn Pro Pro Ile Pro
                245                 250                 255

Val Gly Glu Ile Tyr Lys Arg Trp Ile Ile Leu Gly Leu Asn Lys Ile
            260                 265                 270

Val Arg Met Tyr Ser Pro Thr Ser Ile Leu Asp Ile Arg Gln Gly Pro
        275                 280                 285

Lys Glu Pro Phe Arg Asp Tyr Val Asp Arg Phe Tyr Lys Thr Leu Arg
    290                 295                 300

Ala Glu Gln Ala Ser Gln Glu Val Lys Asn Trp Met Thr Glu Thr Leu
305                 310                 315                 320

Leu Val Gln Asn Ala Asn Pro Asp Cys Lys Thr Ile Leu Lys Ala Leu
                325                 330                 335

Gly Pro Ala Ala Thr Leu Glu Glu Met Met Thr Ala Cys Gln Gly Val
            340                 345                 350

Gly Gly Pro Gly His Lys Ala Arg Val Leu Ala Glu Ala Met Ser Gln
        355                 360                 365

Val Thr Asn Ser Ala Thr Ile Met Met Gln Arg Gly Asn Phe Arg Asn
    370                 375                 380

Gln Arg Lys Ile Val Lys Cys Phe Asn Cys Gly Lys Glu Gly His Thr
385                 390                 395                 400

Ala Arg Asn Cys Arg Ala Pro Arg Lys Lys Gly Cys Trp Lys Cys Gly
                405                 410                 415

Lys Glu Gly His Gln Met Lys Asp Cys Thr Glu Arg Gln Ala Asn Phe
            420                 425                 430

Leu Gly Lys Ile Trp Pro Ser Tyr Lys Gly Arg Pro Gly Asn Phe Leu
        435                 440                 445

Gln Ser Arg Pro Glu Pro Thr Ala Pro Pro Glu Glu Ser Phe Arg Ser
    450                 455                 460

Gly Val Glu Thr Thr Thr Pro Pro Gln Lys Gln Glu Pro Ile Asp Lys
465                 470                 475                 480

Glu Leu Tyr Pro Leu Thr Ser Leu Arg Ser Leu Phe Gly Asn Asp Pro
                485                 490                 495

Ser Ser Gln

<210>  3
<211>  1497
<212>  DNA
<213>  Artificial Sequence

<220>
<223>  Nucleotide sequence coding for gag polypeptide lacking the
       myristoylation sequence and the first methionine

<400>  3
ggtgcgagag cgtcagtatt aagcggggga gaattagatc gatgggaaaa aattcggtta      60

aggccagggg gaaagaagaa gtacaagcta aagcacatcg tatgggcaag cagggagcta     120

gaacgattcg cagttaatcc tggcctgtta gaaacatcag aaggctgtag acaaatactg     180

ggacagctac aaccatccct tcagacagga tcagaggagc ttcgatcact atacaacaca     240

gtagcaaccc tctattgtgt gcaccagcgg atcgagatca aggacaccaa ggaagcttta     300

gacaagatag aggaagagca aaacaagtcc aagaagaagg cccagcaggc agcagctgac     360

acaggacaca gcaatcaggt cagccaaaat taccctatag tgcagaacat ccaggggcaa     420

atggtacatc aggccatatc acctagaact ttaaatgcat gggtaaaagt agtagaagag     480

aaggctttca gcccagaagt gatacccatg ttttcagcat tatcagaagg agccacccca     540

caggacctga acacgatgtt gaacaccgtg gggggacatc aagcagccat gcaaatgtta 600

aaagagacca tcaatgagga agctgcagaa tgggatagag tgcatccagt gcatgcaggg 660

cctattgcac caggccagat gagagaacca aggggaagtg acatagcagg aactactagt 720

acccttcagg aacaaatagg atggatgaca aataatccac ctatcccagt aggagagatc 780

tacaagaggt ggataatcct gggattgaac aagatcgtga ggatgtatag ccctaccagc 840

attctggaca taagacaagg accaaaagaa ccctttagag actatgtaga ccggttctat 900

aaaactctaa gagctgagca agcttcacag gaggtaaaaa attggatgac agaaaccttg 960

ttggtccaaa atgcgaaccc agattgtaag accatcctga aggctctcgg cccagcggct 1020

acactagaag aaatgatgac agcatgtcag ggagtaggag gacccggcca taaggcaaga 1080

gttttggctg aagcaatgag ccaagtaaca aattcagcta ccataatgat gcagagaggc 1140

aattttagga accaaagaaa gattgttaag tgtttcaatt gtggcaaaga agggcacaca 1200

gccagaaatt gcagggcccc taggaaaaag ggctgttgga aatgtggaaa ggaaggacac 1260

caaatgaaag attgtactga gagacaggct aattttttag ggaagatctg gccttcctac 1320

aagggaaggc cagggaattt tcttcagagc agaccagagc caacagcccc accagaagag 1380

agcttcaggt ctggggtaga gacaacaact ccccctcaga agcaggagcc gatagacaag 1440

gaactgtatc ctttaacttc cctcagatca ctctttggca acgacccctc gtcacaa 1497

<210> 4
<211> 263
<212> DNA
<213> Artificial Sequence

<220>
<223> Nucleotide sequence of the Min protein gp41 variant (HR2+
 MPER+TM)

<400> 4
atgatttgga ataacatgac ctggatggag tgggacagag aaattaacaa ttacacaagc 60

ttaatacact ccttaattga agaatcgcaa aaccagcaag aaaagaatga acaagaatta 120

ttggaattag ataaatgggc aagtttgtgg aattggttta acataacaaa ttggctgtgg 180

tacataaaat tattcataat gatagtagga ggcttggtag gtttaagaat agtttttgct 240

gtactttcta tagtgaatag agc 263

<210> 5
<211> 63
<212> PRT
<213> Artificial Sequence

<220>
<223> Amino acid sequence of the gp41 variant (HR2+ MPER)

<400>  5

Met Ile Trp Asn Asn Met Thr Trp Met Glu Trp Asp Arg Glu Ile Asn
1               5                   10                  15

Asn Tyr Thr Ser Leu Ile His Ser Leu Ile Glu Glu Ser Gln Asn Gln
            20                  25                  30

Gln Glu Lys Asn Glu Gln Glu Leu Leu Glu Leu Asp Lys Trp Ala Ser
        35                  40                  45

Leu Trp Asn Trp Phe Asn Ile Thr Asn Trp Leu Trp Tyr Ile Lys
    50                  55                  60

<210>  6
<211>  12
<212>  DNA
<213>  Artificial Sequence

<220>
<223>  Linker Glycine

<400>  6
ggtggcggtg gc                                                          12

<210>  7
<211>  27
<212>  DNA
<213>  Artificial Sequence

<220>
<223>  Partial polylinker of pcDNA 3.1D/V5-His-TOPO® (invitrogen)

<400>  7
aagggtcaag acaattctgc agatatc                                          27

<210>  8
<211>  108
<212>  DNA
<213>  Artificial Sequence

<220>
<223>  Vector sequence that include the V5-epitope, the 6xHis tag and
       stop codon

<400>  8
ggcggccgct cgagtctaga gggcccgcgg ttcgaaggta agcctatccc taaccctctc      60

ctcggtctcg attctacgcg taccggtcat catcaccatc accattga                  108

<210>  9
<211>  1908
<212>  PRT
<213>  Artificial Sequence

<220>

<223>  Nucleotide sequence of the fusion protein (MINGAG3.7)

<400>  9
   Ala Thr Gly Ala Thr Thr Thr Gly Gly Ala Ala Thr Ala Ala Cys Ala
   1               5                   10                  15
   Thr Gly Ala Cys Cys Thr Gly Gly Ala Thr Gly Gly Ala Gly Thr Gly
               20                  25                  30
   Gly Gly Ala Cys Ala Gly Ala Gly Ala Ala Ala Thr Thr Ala Ala Cys
           35                  40                  45
   Ala Ala Thr Thr Ala Cys Ala Cys Ala Ala Gly Cys Thr Thr Ala Ala
       50                  55                  60
   Thr Ala Cys Ala Cys Thr Cys Cys Thr Thr Ala Ala Thr Thr Gly Ala
   65                  70                  75                  80
   Ala Gly Ala Ala Thr Cys Gly Cys Ala Ala Ala Ala Cys Cys Ala Gly
                   85                  90                  95
   Cys Ala Ala Gly Ala Ala Ala Ala Gly Ala Ala Thr Gly Ala Ala Cys
               100                 105                 110
   Ala Ala Gly Ala Ala Thr Thr Ala Thr Thr Gly Gly Ala Ala Thr Thr
           115                 120                 125
   Ala Gly Ala Thr Ala Ala Ala Thr Gly Gly Gly Cys Ala Ala Gly Thr
       130                 135                 140
   Thr Thr Gly Thr Gly Gly Ala Ala Thr Thr Gly Gly Thr Thr Thr Ala
   145                 150                 155                 160
   Ala Cys Ala Thr Ala Ala Cys Ala Ala Ala Thr Thr Gly Gly Cys Thr
                   165                 170                 175
   Gly Thr Gly Gly Thr Ala Cys Ala Thr Ala Ala Ala Ala Thr Thr Ala
               180                 185                 190
   Thr Thr Cys Ala Thr Ala Ala Thr Gly Ala Thr Ala Gly Thr Ala Gly
           195                 200                 205
   Gly Ala Gly Gly Cys Thr Thr Gly Gly Thr Ala Gly Gly Thr Thr Thr
       210                 215                 220
   Ala Ala Gly Ala Ala Thr Ala Gly Thr Thr Thr Thr Thr Gly Cys Thr
   225                 230                 235                 240
   Gly Thr Ala Cys Thr Thr Thr Cys Thr Ala Thr Ala Gly Thr Gly Ala
                   245                 250                 255
   Ala Thr Ala Gly Ala Gly Cys Thr Gly Gly Thr Gly Gly Cys Gly Gly
               260                 265                 270
   Thr Gly Gly Cys Ala Ala Gly Gly Gly Thr Cys Ala Ala Gly Ala Cys
           275                 280                 285
   Ala Ala Thr Thr Cys Thr Gly Cys Ala Gly Ala Thr Ala Thr Cys Gly
       290                 295                 300
   Gly Thr Gly Cys Gly Ala Gly Ala Gly Cys Gly Thr Cys Ala Gly Thr
   305                 310                 315                 320
   Ala Thr Thr Ala Ala Gly Cys Gly Gly Gly Gly Gly Ala Gly Ala Ala
                   325                 330                 335
   Thr Thr Ala Gly Ala Thr Cys Gly Ala Thr Gly Gly Gly Gly Ala Ala
               340                 345                 350
   Ala Ala Ala Thr Thr Cys Gly Gly Thr Thr Ala Ala Gly Gly Cys Cys
           355                 360                 365
   Ala Gly Gly Gly Gly Gly Ala Ala Ala Gly Ala Ala Gly Ala Ala Gly
       370                 375                 380
   Thr Ala Cys Ala Ala Gly Cys Thr Ala Ala Ala Gly Cys Ala Cys Ala
   385                 390                 395                 400
   Thr Cys Gly Thr Ala Thr Gly Gly Gly Cys Ala Ala Gly Cys Ala Gly
                   405                 410                 415
   Gly Gly Ala Gly Cys Thr Ala Gly Ala Ala Cys Gly Ala Thr Thr Cys
               420                 425                 430
   Gly Cys Ala Gly Thr Thr Ala Ala Thr Cys Cys Thr Gly Gly Cys Cys
           435                 440                 445
   Thr Gly Thr Thr Ala Gly Ala Ala Ala Cys Ala Thr Cys Ala Gly Ala
       450                 455                 460
   Ala Gly Gly Cys Thr Gly Thr Ala Gly Ala Cys Ala Ala Ala Thr Ala
   465                 470                 475                 480
   Cys Thr Gly Gly Gly Ala Cys Ala Gly Cys Thr Ala Cys Ala Ala Cys
                   485                 490                 495
   Cys Ala Thr Cys Cys Cys Thr Thr Cys Ala Gly Ala Cys Ala Gly Gly
                   500                 505                 510
   Ala Thr Cys Ala Gly Ala Gly Gly Ala Gly Cys Thr Thr Cys Gly Ala
           515                 520                 525
   Thr Cys Ala Cys Thr Ala Thr Ala Cys Ala Ala Cys Ala Cys Ala Gly
       530                 535                 540
   Thr Ala Gly Cys Ala Ala Cys Cys Cys Thr Cys Thr Ala Thr Thr Gly
   545                 550                 555                 560
   Thr Gly Thr Gly Cys Ala Cys Cys Ala Gly Cys Gly Gly Ala Thr Cys
                   565                 570                 575
   Gly Ala Gly Ala Thr Cys Ala Ala Gly Gly Ala Cys Ala Cys Cys Ala
                   580                 585                 590
   Ala Gly Gly Ala Ala Gly Cys Thr Thr Thr Ala Gly Ala Cys Ala Ala
           595                 600                 605
   Gly Ala Thr Ala Gly Ala Gly Gly Ala Ala Gly Ala Gly Cys Ala Ala
       610                 615                 620
   Ala Ala Cys Ala Ala Gly Thr Cys Cys Ala Ala Gly Ala Ala Gly Ala
   625                 630                 635                 640
   Ala Gly Gly Cys Cys Cys Ala Gly Cys Ala Gly Gly Cys Ala Gly Cys
                   645                 650                 655
   Ala Gly Cys Thr Gly Ala Cys Ala Cys Ala Gly Gly Ala Cys Ala Cys
                   660                 665                 670
   Ala Gly Cys Ala Ala Thr Cys Ala Gly Gly Thr Cys Ala Gly Cys Cys
           675                 680                 685
   Ala Ala Ala Ala Thr Thr Ala Cys Cys Cys Thr Ala Thr Ala Gly Thr
       690                 695                 700
   Gly Cys Ala Gly Ala Ala Cys Ala Thr Cys Cys Ala Gly Gly Gly Gly
   705                 710                 715                 720
   Cys Ala Ala Ala Thr Gly Gly Thr Ala Cys Ala Thr Cys Ala Gly Gly
                   725                 730                 735
   Cys Cys Ala Thr Ala Thr Cys Ala Cys Cys Thr Ala Gly Ala Ala Cys
   740 745 750
   Thr Thr Thr Ala Ala Ala Thr Gly Cys Ala Thr Gly Gly Gly Thr Ala
   755 760 765
   Ala Ala Ala Gly Thr Ala Gly Thr Ala Gly Ala Ala Gly Ala Gly Ala
   770 775 780
   Ala Gly Gly Cys Thr Thr Thr Cys Ala Gly Cys Cys Cys Ala Gly Ala
   785 790 795 800
   Ala Gly Thr Gly Ala Thr Ala Cys Cys Cys Ala Thr Gly Thr Thr Thr
   805 810 815
   Thr Cys Ala Gly Cys Ala Thr Thr Ala Thr Cys Ala Gly Ala Ala Gly
   820 825 830
   Gly Ala Gly Cys Cys Ala Cys Cys Cys Cys Ala Cys Ala Gly Gly Ala
   835 840 845
   Cys Cys Thr Gly Ala Ala Cys Ala Cys Gly Ala Thr Gly Thr Thr Gly
   850 855 860
   Ala Ala Cys Ala Cys Cys Gly Thr Gly Gly Gly Gly Gly Gly Ala Cys
   865 870 875 880
   Ala Thr Cys Ala Ala Gly Cys Ala Gly Cys Cys Ala Thr Gly Cys Ala
   885 890 895
   Ala Ala Thr Gly Thr Thr Ala Ala Ala Ala Gly Ala Gly Ala Cys Cys
   900 905 910
   Ala Thr Cys Ala Ala Thr Gly Ala Gly Gly Ala Ala Gly Cys Thr Gly
   915 920 925
   Cys Ala Gly Ala Ala Thr Gly Gly Gly Ala Thr Ala Gly Ala Gly Thr
   930 935 940
   Gly Cys Ala Thr Cys Cys Ala Gly Thr Gly Cys Ala Thr Gly Cys Ala
   945 950 955 960
   Gly Gly Gly Cys Cys Thr Ala Thr Thr Gly Cys Ala Cys Cys Ala Gly
   965 970 975
   Gly Cys Cys Ala Gly Ala Thr Gly Ala Gly Ala Gly Ala Ala Cys Cys
   980 985 990
   Ala Ala Gly Gly Gly Gly Ala Ala  Gly Thr Gly Ala Cys  Ala Thr Ala
           995                 1000                1005
   Gly Cys  Ala Gly Gly Ala Ala  Cys Thr Ala Cys Thr  Ala Gly Thr
       1010                 1015                 1020
   Ala Cys  Cys Cys Thr Thr Cys  Ala Gly Gly Ala Ala  Cys Ala Ala
       1025                 1030                 1035
   Ala Thr  Ala Gly Gly Ala Thr  Gly Gly Ala Thr Gly  Ala Cys Ala
       1040                 1045                 1050
   Ala Ala  Thr Ala Ala Thr Cys  Cys Ala Cys Cys Thr  Ala Thr Cys
       1055                 1060                 1065
   Cys Cys  Ala Gly Thr Ala Gly  Gly Ala Gly Ala Gly  Ala Thr Cys
       1070                 1075                 1080
   Thr Ala  Cys Ala Ala Gly Ala  Gly Gly Thr Gly Gly  Ala Thr Ala
       1085                 1090                 1095
   Ala Thr  Cys Cys Thr Gly Gly  Gly Ala Thr Thr Gly  Ala Ala Cys
       1100                 1105                 1110
   Ala Ala  Gly Ala Thr Cys Gly  Thr Gly Ala Gly Gly  Ala Thr Gly
       1115                 1120                 1125
   Thr Ala  Thr Ala Gly Cys Cys  Cys Thr Ala Cys Cys  Ala Gly Cys
       1130                 1135                 1140
   Ala Thr  Thr Cys Thr Gly Gly  Ala Cys Ala Thr Ala  Ala Gly Ala
       1145                 1150                 1155
   Cys Ala  Ala Gly Gly Ala Cys  Cys Ala Ala Ala Ala  Gly Ala Ala
       1160                 1165                 1170
   Cys Cys  Cys Thr Thr Thr Ala  Gly Ala Gly Ala Cys  Thr Ala Thr
       1175                 1180                 1185
   Gly Thr  Ala Gly Ala Cys Cys  Gly Gly Thr Thr Cys  Thr Ala Thr
       1190                 1195                 1200
   Ala Ala  Ala Ala Cys Thr Cys  Thr Ala Ala Gly Ala  Gly Cys Thr
       1205                 1210                 1215
   Gly Ala  Gly Cys Ala Ala Gly  Cys Thr Thr Cys Ala  Cys Ala Gly
       1220                 1225                 1230
   Gly Ala Gly Gly Thr Ala Ala Ala Ala Ala Ala Thr Thr Gly Gly
   1235 1240 1245
   Ala Thr Gly Ala Cys Ala Gly Ala Ala Ala Cys Cys Thr Thr Gly
   1250 1255 1260
   Thr Thr Gly Gly Thr Cys Cys Ala Ala Ala Ala Thr Gly Cys Gly
   1265 1270 1275
   Ala Ala Cys Cys Cys Ala Gly Ala Thr Thr Gly Thr Ala Ala Gly
   1280 1285 1290
   Ala Cys Cys Ala Thr Cys Cys Thr Gly Ala Ala Gly Gly Cys Thr
   1295 1300 1305
   Cys Thr Cys Gly Gly Cys Cys Cys Ala Gly Cys Gly Gly Cys Thr
   1310 1315 1320
   Ala Cys Ala Cys Thr Ala Gly Ala Ala Gly Ala Ala Ala Thr Gly
   1325 1330 1335
   Ala Thr Gly Ala Cys Ala Gly Cys Ala Thr Gly Thr Cys Ala Gly
   1340 1345 1350
   Gly Gly Ala Gly Thr Ala Gly Gly Ala Gly Gly Ala Cys Cys Cys
   1355 1360 1365
   Gly Gly Cys Cys Ala Thr Ala Ala Gly Gly Cys Ala Ala Gly Ala
   1370 1375 1380
   Gly Thr Thr Thr Thr Gly Gly Cys Thr Gly Ala Ala Gly Cys Ala
   1385 1390 1395
   Ala Thr Gly Ala Gly Cys Cys Ala Ala Gly Thr Ala Ala Cys Ala
   1400 1405 1410
   Ala Ala Thr Thr Cys Ala Gly Cys Thr Ala Cys Cys Ala Thr Ala
   1415 1420 1425
   Ala Thr Gly Ala Thr Gly Cys Ala Gly Ala Gly Ala Gly Gly Cys
   1430 1435 1440
   Ala Ala Thr Thr Thr Thr Ala Gly Gly Ala Ala Cys Cys Ala Ala
   1445 1450 1455
   Ala Gly Ala Ala Ala Gly Ala Thr Thr Gly Thr Thr Ala Ala Gly
   1460 1465 1470
   Thr Gly Thr Thr Thr Cys Ala Ala Thr Thr Gly Thr Gly Gly Cys
       1475                1480                1485
   Ala Ala Ala Gly Ala Ala Gly Gly Gly Cys Ala Cys Ala Cys Ala
       1490                1495                1500
   Gly Cys Cys Ala Gly Ala Ala Ala Thr Thr Gly Cys Ala Gly Gly
       1505                1510                1515
   Gly Cys Cys Cys Cys Thr Ala Gly Gly Ala Ala Ala Ala Ala Gly
       1520                1525                1530
   Gly Gly Cys Thr Gly Thr Thr Gly Gly Ala Ala Ala Thr Gly Thr
       1535                1540                1545
   Gly Gly Ala Ala Ala Gly Gly Ala Ala Gly Gly Ala Cys Ala Cys
       1550                1555                1560
   Cys Ala Ala Ala Thr Gly Ala Ala Ala Gly Ala Thr Thr Gly Thr
       1565                1570                1575
   Ala Cys Thr Gly Ala Gly Ala Gly Ala Cys Ala Gly Gly Cys Thr
       1580                1585                1590
   Ala Ala Thr Thr Thr Thr Thr Thr Ala Gly Gly Gly Ala Ala Gly
       1595                1600                1605
   Ala Thr Cys Thr Gly Gly Cys Cys Thr Thr Cys Cys Thr Ala Cys
       1610                1615                1620
   Ala Ala Gly Gly Gly Ala Ala Gly Gly Cys Cys Ala Gly Gly Gly
       1625                1630                1635
   Ala Ala Thr Thr Thr Thr Cys Thr Thr Cys Ala Gly Ala Gly Cys
       1640                1645                1650
   Ala Gly Ala Cys Cys Ala Gly Ala Gly Cys Cys Ala Ala Cys Ala
       1655                1660                1665
   Gly Cys Cys Cys Cys Ala Cys Cys Ala Gly Ala Ala Gly Ala Gly
       1670                1675                1680
   Ala Gly Cys Thr Thr Cys Ala Gly Gly Thr Cys Thr Gly Gly Gly
       1685                1690                1695
   Gly Thr Ala Gly Ala Gly Ala Cys Ala Ala Cys Ala Ala Cys Thr
   1700                    1705                    1710
   Cys Cys  Cys Cys Cys Thr Cys  Ala Gly Ala Ala Gly  Cys Ala Gly
       1715                    1720                    1725
   Gly Ala  Gly Cys Cys Gly Ala  Thr Ala Gly Ala Cys  Ala Ala Gly
       1730                    1735                    1740
   Gly Ala  Ala Cys Thr Gly Thr  Ala Thr Cys Cys Thr  Thr Thr Ala
       1745                    1750                    1755
   Ala Cys  Thr Thr Cys Cys Cys  Thr Cys Ala Gly Ala  Thr Cys Ala
       1760                    1765                    1770
   Cys Thr  Cys Thr Thr Thr Gly  Gly Cys Ala Ala Cys  Gly Ala Cys
       1775                    1780                    1785
   Cys Cys  Cys Thr Cys Gly Thr  Cys Ala Cys Ala Ala  Gly Gly Cys
       1790                    1795                    1800
   Gly Gly  Cys Cys Gly Cys Thr  Cys Gly Ala Gly Thr  Cys Thr Ala
       1805                    1810                    1815
   Gly Ala  Gly Gly Gly Cys Cys  Cys Gly Cys Gly Gly  Thr Thr Cys
       1820                    1825                    1830
   Gly Ala  Ala Gly Gly Thr Ala  Ala Gly Cys Cys Thr  Ala Thr Cys
       1835                    1840                    1845
   Cys Cys  Thr Ala Ala Cys Cys  Cys Thr Cys Thr Cys  Cys Thr Cys
       1850                    1855                    1860
   Gly Gly  Thr Cys Thr Cys Gly  Ala Thr Thr Cys Thr  Ala Cys Gly
       1865                    1870                    1875
   Cys Gly  Thr Ala Cys Cys Gly  Gly Thr Cys Ala Thr  Cys Ala Thr
       1880                    1885                    1890
   Cys Ala  Cys Cys Ala Thr Cys  Ala Cys Cys Ala Thr  Thr Gly Ala
       1895                    1900                    1905

<210>  10
<211>  50
<212>  DNA
<213>  Artificial Sequence

<220>
<223>  Sequence of GM-CSF signal peptide

<400>  10
atgtggctgc agtctctgct gctgctgggc accgtggcct gcagcatcag                50


<210>  11
<211>  259
<212>  DNA
<213>  Artificial Sequence

<220>
<223>  Nucleotide sequence encoding the MIN protein (HR2+ MPER+TM)

<400>  11
catctggaac cacaccacct ggatggaatg ggacagagag atcaacaact acaccagcct        60

gatccactcc ctgatcgagg aaagccagaa ccagcaggaa aagaacgaac aggaactgct       120

ggaactggac aagtgggcca gcctgtggaa ctggttcaac atcaccaact ggctgtggta       180

catcaagctg ttcatcatga tcgtgggcgg cctcgtgggc ctgagaatcg tgtttgccgt       240

gctgagcatc gtgaaccgg                                                    259


<210>  12
<211>  15
<212>  DNA
<213>  Artificial Sequence

<220>
<223>  Nucleotide sequence  of linker GS

<400>  12
ggaggcggag gatcc                                                         15


<210>  13
<211>  1497
<212>  DNA
<213>  Artificial Sequence

<220>
<223>  Nucleotide sequence of GAGHXB2cop, codon optimized

<400>  13
ggcgctagag cttctgtgct gtctgggggc gaactggata gatgggagaa gatccggctg        60

cggcctggcg gcaagaagaa gtacaagctg aagcacatcg tgtgggcctc cagagagctg       120

gaaagattcg ccgtgaaccc cggcctgctg gaaaccagcg agggctgcag acagatcctg       180

ggacagctgc agcccagcct gcagaccgga agcgaggaac tgcggagcct gtacaacacc       240

gtggccacac tgtattgcgt gcaccagcgg atcgagatca aggacaccaa agaggccctg       300

gacaagatcg aggaagaaca gaacaagtct aagaagaagg cccagcaggc tgccgccgat       360

accggccact ctaatcaggt gtcccagaac taccccatcg tgcagaacat ccagggccag       420

atggtgcatc aggccatcag ccccagaacc ctgaacgcct gggtcaaggt ggtggaagag       480

aaggccttta gccccgaagt gatccccatg ttcagcgccc tgtctgaggg cgccacaccc       540

caggacctga acaccatgct gaacacagtg ggcggacacc aggccgccat gcagatgctg 600

aaagagacaa tcaacgaaga ggccgccgag tgggacagag tgcaccctgt gcatgccgga 660

cctatcgccc ctggccagat gagagagccc agaggctctg atatcgccgg caccaccagc 720

accctgcagg aacagatcgg ctggatgacc aacaaccccc ccatccccgt gggcgagatc 780

tacaagcggt ggatcatcct gggcctgaac aagattgtgc ggatgtacag ccccacctcc 840

atcctggaca tccggcaggg ccccaaagag cccttcagag actacgtgga ccggttctac 900

aagaccctga gagccgagca ggccagccag gaagtgaaga attggatgac cgagacactg 960

ctggtgcaga acgccaaccc cgactgcaag accatcctga aggccctggg acctgccgcc 1020

accctggaag agatgatgac agcctgtcag ggcgtgggag gcccaggcca caaagctaga 1080

gtgctggccg aggccatgag ccaagtgacc aactccgcca ccattatgat gcagcggggc 1140

aacttccgga accagcggaa gatcgtgaag tgcttcaact gcggcaaaga gggccacacc 1200

gcccggaatt gcagagcccc cagaaagaaa ggctgctgga agtgtggaaa agaggggcac 1260

cagatgaagg actgcaccga gcggcaggcc aacttcctgg gcaagatctg gcctagctac 1320

aagggcagac ccggcaattt cctgcagagc agacccgagc ctaccgcccc tcccgaggaa 1380

agctttagaa gcggcgtgga aaccaccacc cccccacaga agcaggaacc catcgacaaa 1440

gagctgtacc ccctgaccag cctgagaagc ctgttcggca acgaccccag cagccag 1497

<210> 14
<211> 6
<212> DNA
<213> Artificial Sequence

<220>
<223> Stop codon

<400> 14
tgatga 6

<210> 15
<211> 1827
<212> DNA
<213> Artificial Sequence

<220>
<223> Nucleotide sequence of MINGAGHXB2cop

<400> 15
atgtggctgc agtctctgct gctgctgggc accgtggcct gcagcatcag catctggaac 60

cacaccacct ggatggaatg ggacagagag atcaacaact acaccagcct gatccactcc 120

ctgatcgagg aaagccagaa ccagcaggaa aagaacgaac aggaactgct ggaactggac 180

aagtgggcca gcctgtggaa ctggttcaac atcaccaact ggctgtggta catcaagctg 240

ttcatcatga tcgtgggcgg cctcgtgggc ctgagaatcg tgtttgccgt gctgagcatc 300

gtgaaccggg gaggcggagg atccggcgct agagcttctg tgctgtctgg gggcgaactg 360

gatagatggg agaagatccg gctgcggcct ggcggcaaga agaagtacaa gctgaagcac 420

atcgtgtggg cctccagaga gctggaaaga ttcgccgtga accccggcct gctggaaacc 480

agcgagggct gcagacagat cctgggacag ctgcagccca gcctgcagac cggaagcgag 540

gaactgcgga gcctgtacaa caccgtggcc acactgtatt gcgtgcacca gcggatcgag 600

atcaaggaca ccaaagaggc cctggacaag atcgaggaag aacagaacaa gtctaagaag 660

aaggcccagc aggctgccgc cgataccggc cactctaatc aggtgtccca gaactacccc 720

atcgtgcaga acatccaggg ccagatggtg catcaggcca tcagccccag aaccctgaac 780

gcctgggtca aggtggtgga agagaaggcc tttagccccg aagtgatccc catgttcagc 840

gccctgtctg agggcgccac accccaggac ctgaacacca tgctgaacac agtgggcgga 900

caccaggccg ccatgcagat gctgaaagag acaatcaacg aagaggccgc cgagtgggac 960

agagtgcacc ctgtgcatgc cggacctatc gcccctggcc agatgagaga gcccagaggc 1020

tctgatatcg ccggcaccac cagcaccctg caggaacaga tcggctggat gaccaacaac 1080

ccccccatcc ccgtgggcga gatctacaag cggtggatca tcctgggcct gaacaagatt 1140

gtgcggatgt acagccccac ctccatcctg gacatccggc agggccccaa agagcccttc 1200

agagactacg tggaccggtt ctacaagacc ctgagagccg agcaggccag ccaggaagtg 1260

aagaattgga tgaccgagac actgctggtg cagaacgcca accccgactg caagaccatc 1320

ctgaaggccc tgggacctgc cgccaccctg gaagagatga tgacagcctg tcagggcgtg 1380

ggaggcccag gccacaaagc tagagtgctg gccgaggcca tgagccaagt gaccaactcc 1440

gccaccatta tgatgcagcg gggcaacttc cggaaccagc ggaagatcgt gaagtgcttc 1500

aactgcggca aagagggcca caccgcccgg aattgcagag cccccagaaa gaaaggctgc 1560

tggaagtgtg gaaaagaggg gcaccagatg aaggactgca ccgagcggca ggccaacttc 1620

ctgggcaaga tctggcctag ctacaagggc agacccggca atttcctgca gagcagaccc 1680

gagcctaccg cccctcccga ggaaagcttt agaagcggcg tggaaaccac caccccccca 1740

cagaagcagg aacccatcga caaagagctg taccccctga ccagcctgag aagcctgttc 1800

ggcaacgacc ccagcagcca gtgatga 1827

<210> 16
<211> 173
<212> PRT
<213> Artificial Sequence

<220>
<223> Amino acid sequence of the extracellular region of gp41

<400> 16

Ala Val Gly Ile Gly Ala Leu Phe Leu Gly Phe Leu Gly Ala Ala Gly
1               5                   10                  15

Ser Thr Met Gly Ala Ala Ser Met Thr Leu Thr Val Gln Ala Arg Gln
            20                  25                  30

Leu Leu Ser Gly Ile Val Gln Gln Gln Asn Asn Leu Leu Arg Ala Ile
        35                  40                  45

Glu Ala Gln Gln His Leu Leu Gln Leu Thr Val Trp Gly Ile Lys Gln
    50                  55                  60

Leu Gln Ala Arg Ile Leu Ala Val Glu Arg Tyr Leu Lys Asp Gln Gln
65                  70                  75                  80

Leu Leu Gly Ile Trp Gly Cys Ser Gly Lys Leu Ile Cys Thr Thr Ala
                85                  90                  95

Val Pro Trp Asn Ala Ser Trp Ser Asn Lys Ser Leu Glu Gln Ile Trp
            100                 105                 110

Asn His Thr Thr Trp Met Glu Trp Asp Arg Glu Ile Asn Asn Tyr Thr
        115                 120                 125

Ser Leu Ile His Ser Leu Ile Glu Glu Ser Gln Asn Gln Gln Glu Lys
    130                 135                 140

Asn Glu Gln Glu Leu Leu Glu Leu Asp Lys Trp Ala Ser Leu Trp Asn
145                 150                 155                 160

Trp Phe Asn Ile Thr Asn Trp Leu Trp Tyr Ile Lys Leu
                165                 170

<210>  17
<211>  24
<212>  PRT
<213>  Artificial Sequence

<220>
<223>  Amino acid sequence of the transmembrane domain of gp41

<400>  17

Leu Phe Ile Met Ile Val Gly Gly Leu Val Gly Leu Arg Ile Val Phe
1               5                   10                  15

Ala Val Leu Ser Ile Val Asn Arg
            20

<210>  18
<211>  19
<212>  PRT
<213>  Artificial Sequence

<220>
<223>  amino acid sequence of the transmembrane domain of CD22

<400>  18

Val Ala Val Gly Leu Gly Ser Cys Leu Ala Ile Leu Ile Leu Ala Ile
1               5                   10                  15

Cys Gly Leu

<210>  19
<211>  22
<212>  PRT
<213>  Artificial Sequence

<220>
<223>  Amino acid sequence of the transmembrane domain of CD36

<400>  19

Leu Leu Gly Leu Ile Glu Met Ile Leu Leu Ser Val Gly Val Val Met
1               5                   10                  15

Phe Val Ala Phe Met Ile
            20

<210>  20
<211>  21
<212>  PRT
<213>  Artificial Sequence

<220>
<223>  Amino acid sequence of the transmembrane domain of CD44

<400>  20

Trp Leu Ile Ile Leu Ala Ser Leu Leu Ala Leu Ala Leu Ile Leu Ala
1               5                   10                  15

Val Cys Ile Ala Val
            20

<210>  21
<211>  856
<212>  PRT
<213>  Artificial Sequence

<220>
<223>  Env protein of HIV (isolate HXB2)

<400>  21

Met Arg Val Lys Glu Lys Tyr Gln His Leu Trp Arg Trp Gly Trp Arg
1               5                   10                  15

Trp Gly Thr Met Leu Leu Gly Met Leu Met Ile Cys Ser Ala Thr Glu
            20                  25                  30

Lys Leu Trp Val Thr Val Tyr Tyr Gly Val Pro Val Trp Lys Glu Ala
        35                  40                  45

Thr Thr Thr Leu Phe Cys Ala Ser Asp Ala Lys Ala Tyr Asp Thr Glu
    50                  55                  60

Val His Asn Val Trp Ala Thr His Ala Cys Val Pro Thr Asp Pro Asn
65                  70                  75                  80

Pro Gln Glu Val Val Leu Val Asn Val Thr Glu Asn Phe Asn Met Trp
                85                  90                  95

Lys Asn Asp Met Val Glu Gln Met His Glu Asp Ile Ile Ser Leu Trp
            100                 105                 110

Asp Gln Ser Leu Lys Pro Cys Val Lys Leu Thr Pro Leu Cys Val Ser
        115                 120                 125

Leu Lys Cys Thr Asp Leu Lys Asn Asp Thr Asn Thr Asn Ser Ser Ser
    130                 135                 140

Gly Arg Met Ile Met Glu Lys Gly Glu Ile Lys Asn Cys Ser Phe Asn
145                 150                 155                 160

Ile Ser Thr Ser Ile Arg Gly Lys Val Gln Lys Glu Tyr Ala Phe Phe
                165                 170                 175

Tyr Lys Leu Asp Ile Ile Pro Ile Asp Asn Asp Thr Thr Ser Tyr Lys
            180                 185                 190

Leu Thr Ser Cys Asn Thr Ser Val Ile Thr Gln Ala Cys Pro Lys Val
        195                 200                 205

Ser Phe Glu Pro Ile Pro Ile His Tyr Cys Ala Pro Ala Gly Phe Ala
    210                 215                 220

Ile Leu Lys Cys Asn Asn Lys Thr Phe Asn Gly Thr Gly Pro Cys Thr
225                 230                 235                 240

Asn Val Ser Thr Val Gln Cys Thr His Gly Ile Arg Pro Val Val Ser
                245                 250                 255

Thr Gln Leu Leu Leu Asn Gly Ser Leu Ala Glu Glu Glu Val Val Ile
        260                 265                 270

Arg Ser Val Asn Phe Thr Asp Asn Ala Lys Thr Ile Ile Val Gln Leu
    275                 280                 285

Asn Thr Ser Val Glu Ile Asn Cys Thr Arg Pro Asn Asn Asn Thr Arg
    290                 295                 300

Lys Arg Ile Arg Ile Gln Arg Gly Pro Gly Arg Ala Phe Val Thr Ile
305                 310                 315                 320

Gly Lys Ile Gly Asn Met Arg Gln Ala His Cys Asn Ile Ser Arg Ala
                325                 330                 335

Lys Trp Asn Asn Thr Leu Lys Gln Ile Ala Ser Lys Leu Arg Glu Gln
        340                 345                 350

Phe Gly Asn Asn Lys Thr Ile Ile Phe Lys Gln Ser Ser Gly Gly Asp
    355                 360                 365

Pro Glu Ile Val Thr His Ser Phe Asn Cys Gly Gly Glu Phe Phe Tyr
    370                 375                 380

Cys Asn Ser Thr Gln Leu Phe Asn Ser Thr Trp Phe Asn Ser Thr Trp
385                 390                 395                 400

Ser Thr Glu Gly Ser Asn Asn Thr Glu Gly Ser Asp Thr Ile Thr Leu
                405                 410                 415

Pro Cys Arg Ile Lys Gln Ile Ile Asn Met Trp Gln Lys Val Gly Lys
        420                 425                 430

Ala Met Tyr Ala Pro Pro Ile Ser Gly Gln Ile Arg Cys Ser Ser Asn
    435                 440                 445

Ile Thr Gly Leu Leu Leu Thr Arg Asp Gly Gly Asn Ser Asn Asn Glu
    450                 455                 460

Ser Glu Ile Phe Arg Pro Gly Gly Gly Asp Met Arg Asp Asn Trp Arg
465                 470                 475                 480

Ser Glu Leu Tyr Lys Tyr Lys Val Val Lys Ile Glu Pro Leu Gly Val
                485                 490                 495

Ala Pro Thr Lys Ala Lys Arg Arg Val Val Gln Arg Glu Lys Arg Ala

500                 505                 510

Val Gly Ile Gly Ala Leu Phe Leu Gly Phe Leu Gly Ala Ala Gly Ser
        515                 520                 525

Thr Met Gly Ala Ala Ser Met Thr Leu Thr Val Gln Ala Arg Gln Leu
    530                 535                 540

Leu Ser Gly Ile Val Gln Gln Gln Asn Asn Leu Leu Arg Ala Ile Glu
545                 550                 555                 560

Ala Gln Gln His Leu Leu Gln Leu Thr Val Trp Gly Ile Lys Gln Leu
                565                 570                 575

Gln Ala Arg Ile Leu Ala Val Glu Arg Tyr Leu Lys Asp Gln Gln Leu
            580                 585                 590

Leu Gly Ile Trp Gly Cys Ser Gly Lys Leu Ile Cys Thr Thr Ala Val
        595                 600                 605

Pro Trp Asn Ala Ser Trp Ser Asn Lys Ser Leu Glu Gln Ile Trp Asn
    610                 615                 620

His Thr Thr Trp Met Glu Trp Asp Arg Glu Ile Asn Asn Tyr Thr Ser
625                 630                 635                 640

Leu Ile His Ser Leu Ile Glu Glu Ser Gln Asn Gln Gln Glu Lys Asn
                645                 650                 655

Glu Gln Glu Leu Leu Glu Leu Asp Lys Trp Ala Ser Leu Trp Asn Trp
            660                 665                 670

Phe Asn Ile Thr Asn Trp Leu Trp Tyr Ile Lys Leu Phe Ile Met Ile
        675                 680                 685

Val Gly Gly Leu Val Gly Leu Arg Ile Val Phe Ala Val Leu Ser Ile
    690                 695                 700

Val Asn Arg Val Arg Gln Gly Tyr Ser Pro Leu Ser Phe Gln Thr His
705                 710                 715                 720

Leu Pro Thr Pro Arg Gly Pro Asp Arg Pro Glu Gly Ile Glu Glu Glu
                725                 730                 735

Gly Gly Glu Arg Asp Arg Asp Arg Ser Ile Arg Leu Val Asn Gly Ser
            740                 745                 750

Leu Ala Leu Ile Trp Asp Asp Leu Arg Ser Leu Cys Leu Phe Ser Tyr
        755                 760                 765

His Arg Leu Arg Asp Leu Leu Leu Ile Val Thr Arg Ile Val Glu Leu
    770                 775                 780

Leu Gly Arg Arg Gly Trp Glu Ala Leu Lys Tyr Trp Trp Asn Leu Leu
785                 790                 795                 800

Gln Tyr Trp Ser Gln Glu Leu Lys Asn Ser Ala Val Ser Leu Leu Asn
                805                 810                 815

Ala Thr Ala Ile Ala Val Ala Glu Gly Thr Asp Arg Val Ile Glu Val
            820                 825                 830

Val Gln Gly Ala Cys Arg Ala Ile Arg His Ile Pro Arg Arg Ile Arg
        835                 840                 845

Gln Gly Leu Glu Arg Ile Leu Leu
    850                 855

<210>  22
<211>  41
<212>  PRT
<213>  Artificial Sequence

<220>
<223>  Amino acid sequence of HR2 region

<400>  22

Met Ile Trp Asn Asn Met Thr Trp Met Glu Trp Asp Arg Glu Ile Asn
1               5                   10                  15

Asn Tyr Thr Ser Leu Ile His Ser Leu Ile Glu Glu Ser Gln Asn Gln
            20                  25                  30

Gln Glu Lys Asn Glu Gln Glu Leu Leu
        35                  40

<210>  23
<211>  22
<212>  PRT
<213>  Artificial Sequence

<220>
<223>  Amino acid sequence of MPER region

<400>  23

Glu Leu Asp Lys Trp Ala Ser Leu Trp Asn Trp Phe Asn Ile Thr Asn
1               5                   10                  15

Trp Leu Trp Tyr Ile Lys
            20

<210>  24
<211>  4
<212>  PRT
<213>  Artificial Sequence

<220>
<223>  Linker

<400>  24

Gly Gly Gly Ser
1

<210>  25
<211>  13
<212>  PRT
<213>  Artificial Sequence

<220>
<223>  Linker

<400>  25

Gly Gly Gly Gly Lys Gly Gln Asp Asn Ser Ala Asp Ile
1               5                   10

<210>  26
<211>  12
<212>  PRT
<213>  Artificial Sequence

<220>
<223>  Linker

<400>  26

Gly Ser Ala Gly Ser Ala Ala Gly Ser Gly Glu Phe
1               5                   10

<210>  27
<211>  7
<212>  PRT
<213>  Artificial Sequence

<220>
<223>  Linker

<400>  27

Ala Glu Ala Ala Ala Lys Ala
1               5

<210>  28
<211>  7

<212>  PRT
<213>  Artificial Sequence

<220>
<223>  Linker

<400>  28

Ala Pro Ala Pro Ala Pro Ala
1               5


<210>  29
<211>  635
<212>  PRT
<213>  Artificial Sequence

<220>
<223>  Amino acid sequence of the fusion protein (MINGAG3.7)

<400>  29

Met Ile Trp Asn Asn Met Thr Trp Met Glu Trp Asp Arg Glu Ile Asn
1               5                   10                  15

Asn Tyr Thr Ser Leu Ile His Ser Leu Ile Glu Glu Ser Gln Asn Gln
            20                  25                  30

Gln Glu Lys Asn Glu Gln Glu Leu Leu Glu Leu Asp Lys Trp Ala Ser
        35                  40                  45

Leu Trp Asn Trp Phe Asn Ile Thr Asn Trp Leu Trp Tyr Ile Lys Leu
    50                  55                  60

Phe Ile Met Ile Val Gly Gly Leu Val Gly Leu Arg Ile Val Phe Ala
65                  70                  75                  80

Val Leu Ser Ile Val Asn Arg Ala Gly Gly Gly Gly Lys Gly Gln Asp
                85                  90                  95

Asn Ser Ala Asp Ile Gly Ala Arg Ala Ser Val Leu Ser Gly Gly Glu
            100                 105                 110

Leu Asp Arg Trp Gly Lys Ile Arg Leu Arg Pro Gly Gly Lys Lys Lys
        115                 120                 125

Tyr Lys Leu Lys His Ile Val Trp Ala Ser Arg Glu Leu Glu Arg Phe
    130                 135                 140

Ala Val Asn Pro Gly Leu Leu Glu Thr Ser Glu Gly Cys Arg Gln Ile
145                 150                 155                 160

Leu Gly Gln Leu Gln Pro Ser Leu Gln Thr Gly Ser Glu Glu Leu Arg

                165                 170                 175

Ser Leu Tyr Asn Thr Val Ala Thr Leu Tyr Cys Val His Gln Arg Ile
            180                 185                 190

Glu Ile Lys Asp Thr Lys Glu Ala Leu Asp Lys Ile Glu Glu Glu Gln
        195                 200                 205

Asn Lys Ser Lys Lys Lys Ala Gln Gln Ala Ala Ala Asp Thr Gly His
    210                 215                 220

Ser Asn Gln Val Ser Gln Asn Tyr Pro Ile Val Gln Asn Ile Gln Gly
225                 230                 235                 240

Gln Met Val His Gln Ala Ile Ser Pro Arg Thr Leu Asn Ala Trp Val
                245                 250                 255

Lys Val Val Glu Glu Lys Ala Phe Ser Pro Glu Val Ile Pro Met Phe
            260                 265                 270

Ser Ala Leu Ser Glu Gly Ala Thr Pro Gln Asp Leu Asn Thr Met Leu
        275                 280                 285

Asn Thr Val Gly Gly His Gln Ala Ala Met Gln Met Leu Lys Glu Thr
    290                 295                 300

Ile Asn Glu Glu Ala Ala Glu Trp Asp Arg Val His Pro Val His Ala
305                 310                 315                 320

Gly Pro Ile Ala Pro Gly Gln Met Arg Glu Pro Arg Gly Ser Asp Ile
                325                 330                 335

Ala Gly Thr Thr Ser Thr Leu Gln Glu Gln Ile Gly Trp Met Thr Asn
            340                 345                 350

Asn Pro Pro Ile Pro Val Gly Glu Ile Tyr Lys Arg Trp Ile Ile Leu
        355                 360                 365

Gly Leu Asn Lys Ile Val Arg Met Tyr Ser Pro Thr Ser Ile Leu Asp
    370                 375                 380

Ile Arg Gln Gly Pro Lys Glu Pro Phe Arg Asp Tyr Val Asp Arg Phe
385                 390                 395                 400

Tyr Lys Thr Leu Arg Ala Glu Gln Ala Ser Gln Glu Val Lys Asn Trp
                405                 410                 415

Met Thr Glu Thr Leu Leu Val Gln Asn Ala Asn Pro Asp Cys Lys Thr
            420                 425                 430

Ile Leu Lys Ala Leu Gly Pro Ala Ala Thr Leu Glu Glu Met Met Thr
        435                 440                 445

Ala Cys Gln Gly Val Gly Gly Pro Gly His Lys Ala Arg Val Leu Ala
    450                 455                 460

Glu Ala Met Ser Gln Val Thr Asn Ser Ala Thr Ile Met Met Gln Arg
465                 470                 475                 480

Gly Asn Phe Arg Asn Gln Arg Lys Ile Val Lys Cys Phe Asn Cys Gly
                485                 490                 495

Lys Glu Gly His Thr Ala Arg Asn Cys Arg Ala Pro Arg Lys Lys Gly
            500                 505                 510

Cys Trp Lys Cys Gly Lys Glu Gly His Gln Met Lys Asp Cys Thr Glu
        515                 520                 525

Arg Gln Ala Asn Phe Leu Gly Lys Ile Trp Pro Ser Tyr Lys Gly Arg
    530                 535                 540

Pro Gly Asn Phe Leu Gln Ser Arg Pro Glu Pro Thr Ala Pro Pro Glu
545                 550                 555                 560

Glu Ser Phe Arg Ser Gly Val Glu Thr Thr Thr Pro Pro Gln Lys Gln
                565                 570                 575

Glu Pro Ile Asp Lys Glu Leu Tyr Pro Leu Thr Ser Leu Arg Ser Leu
            580                 585                 590

Phe Gly Asn Asp Pro Ser Ser Gln Gly Gly Arg Ser Ser Leu Glu Gly
        595                 600                 605

Pro Arg Phe Glu Gly Lys Pro Ile Pro Asn Pro Leu Leu Gly Leu Asp
    610                 615                 620

Ser Thr Arg Thr Gly His His His His His His
625                 630                 635

## Description

### FIELD OF THE INVENTION

The present invention belongs to the field of fusion proteins and virus-like particles (VLPs) for medical use in preventing and/or treating an infection.

### BACKGROUND OF THE INVENTION

Virus-like particles (VLP) represent an excellent vaccine platform that has been proven effective against non-enveloped viruses such as the Hepatitis B Virus (HBV), Human Papilloma Virus (HPV) or Hepatitis E Virus (HEV) [Lua LH, et al., Biotechnol Bioeng 2014, 111:425-440]. In these cases, VLPs contain a single capsid protein that is produced in prokaryotic or eukaryotic cell systems to spontaneously form VLP with excellent immunogenic results. The success and versatility of VLP production platforms has prompted the exploration of more complex designs. Some examples could be the generation of VLPs with heterologous antigen presentation by engrafting exogenous sequences in viral capsid proteins [Bryant M, et al. Proc Natl Acad Sci U S A 1990, 87:523-527] and the generation of enveloped VLPs, which contain a lipid bilayer derived from the VLP-producer cell and show higher heterogeneity than non-enveloped VLPs [Visciano ML et al., Vaccine. 2011 Jul 12;29(31):4903-12].

Human immunodeficiency Virus (HIV) vaccine research has also approached the VLP technology. As for other retroviruses, HIV particles are enveloped structures formed by the multimerization of Gag structural proteins on the inner part of plasma membrane of infected cells through the myrystoylation of its N-term end [Bryant M. et al., cited supra]. During the budding process, Gag polymers incorporate genomic viral RNA, different viral enzymes and accessory proteins and recruit cell surface expressed envelope glycoprotein complexes to ensure infectivity. However, it is well known that expression of a retroviral gag gene is sufficient to produce VLP structures lacking infectivity [Visciano ML cited supra, Cervera L. et al J Biotechnol 2013, 166:152-165]. These nude Gag VLPs can be modified either to incorporate proteins or RNAs and act as delivery vectors or to incorporate proteins on their surface to act as vaccine preparations [Visciano ML et al., cited supra].

Over the last years, several groups have developed the production process of enveloped VLP focusing on HIV vaccine research [Visciano ML cited supra, Cervera L. et al cited supra]. However, the optimal generation of enveloped VLPs is not the only obstacle in these approaches. A second major difficulty is the complexity of HIV envelope glycoprotein (Env) [Mao Y, et al, Nat Struct Mol Biol 2012, 19:893-899]. This large glycoprotein contains two subunits gp120 and gp41 that are able to generate neutralizing and non-neutralizing antibodies [Mao Y, et al, cited supra]. Despite multiple approaches to redesign protein mimics that act as immunogens to elicit neutralizing antibodies against Env, few positive results have been achieved [Mao Y, et al, cited supra].

However, despite the efforts made to date, there still exists a continuing need in the art for novel ways of introducing antigen epitopes in VLPs compounds, as well as developing VLPs useful in eliciting an immune response against particular antigen epitopes.

### SUMMARY OF THE INVENTION

In a first aspect, the invention relates to a fusion protein comprising an HIV gag polypeptide or a functionally equivalent variant thereof further and an immunogenic polypeptide, wherein the immunogenic polypeptide is located N-terminal to the HIV gag polypeptide or to the functionally equivalent variant thereof.

In a second aspect, the invention relates to a polynucleotide encoding a fusion protein according to the invention.

In a third aspect, the invention relates to a vector comprising a polynucleotide according to the invention.

In a fourth aspect, the invention relates to a host cell comprising a fusion protein or a vector according to the invention.

In a fifth aspect, the invention relates to a method for preparing a VLP loaded with an immunogenic polypeptide comprising
(i) expressing in a cell a fusion protein according to the invention and
(ii) recovering the VLPs from the extracellular medium.

In a sixth aspect, the invention relates to virus like-particle comprising the fusion protein according to the invention or obtained using a method of the invention.

In a seventh aspect, the invention relates to an immunogenic composition or a vaccine composition comprising an immunogenic amount of a fusion protein of the invention, a polynucleotide of the invention, a vector of the invention, a host cell of the invention or a virus-like particle of the invention.

In an eighth aspect, the invention relates to a fusion protein of the invention, a polynucleotide of the invention, a vector of the invention, a host cell of the invention, a virus-like particle of the invention or an immunogenic composition or a vaccine composition of the invention for use in medicine.

In a ninth aspect, the invention relates to a fusion protein of the invention, a polynucleotide of the invention, a vector of the invention, a host cell of the invention, a virus-like particle of the invention or an immunogenic composition or a vaccine composition of the invention for use in the prevention and/or treatment of a disease caused by an infection in a subject, wherein the infection is by a pathogen which contains the immunogenic polypeptide forming part of the fusion protein or a region thereof

### BRIEF DESCRIPTION OF THE DRAWINGS

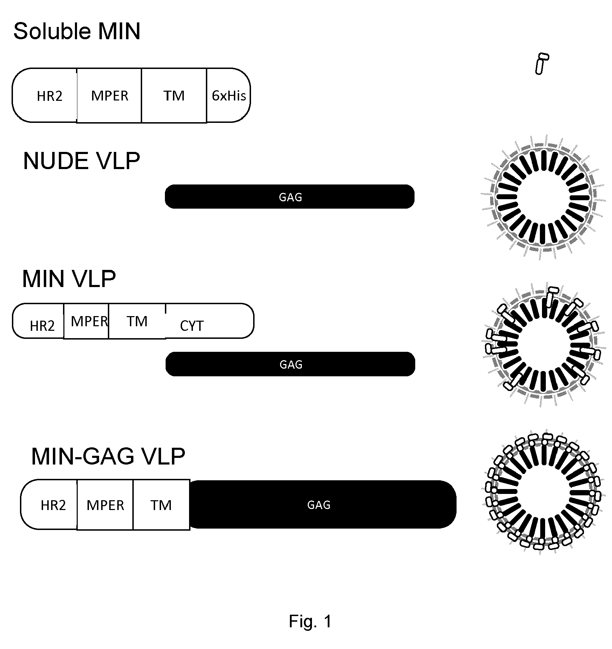

**Figure 1****. Different protein and VLP preparations used for immunogenicity assays.** A gp41-derived protein (MIN) encompassing the HR2, MPER and TM regions of the gp41 glycoprotein was expressed in *E. Coli,* a 6xHis tag was included in the design at the C-terminus of the protein for purification. Nude VLPs were generated by transfection of an HIV-1 gag coding plasmid in 293T cells. These NUDE VLP expose human proteins coming from the producer cell membrane (light grey elements on the surface of VLP). Alternatively, MIN-VLPs were produced by cotransfection of the same gag expressing plasmid with a plasmid coding for the MIN protein that contains the full cytoplasmic tail of gp41. MIN protein is incorporated on the surface of VLPs. A final VLP preparation was produced by expressing a variant of MIN protein lacking the cytoplasmic tail fused to a gag sequence (starting at residue 2). The VLPs formed are expected to display high density MPER epitopes on their surface (gag:gp41 stoichiometry 1:1).

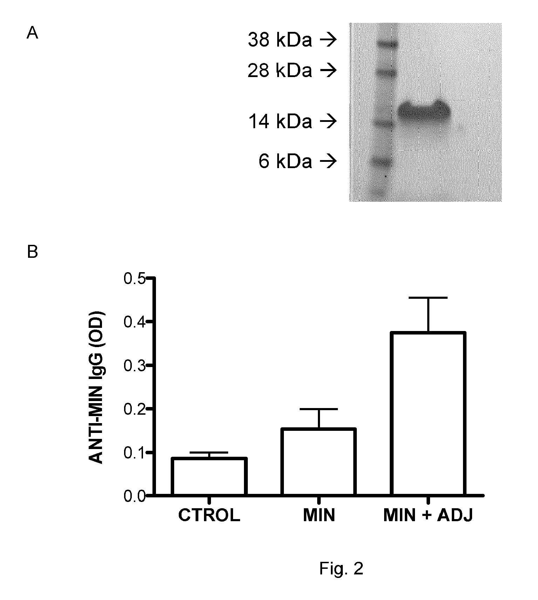

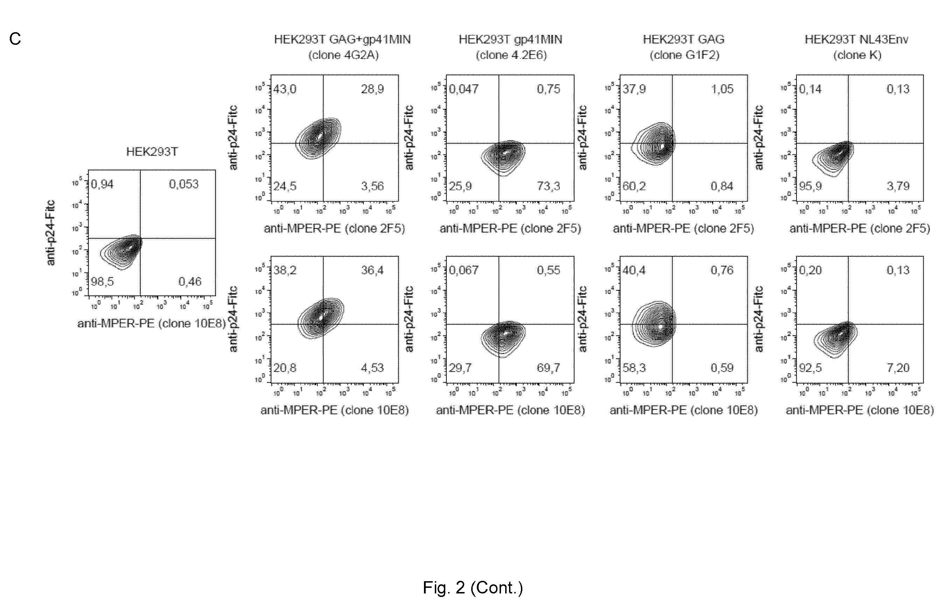

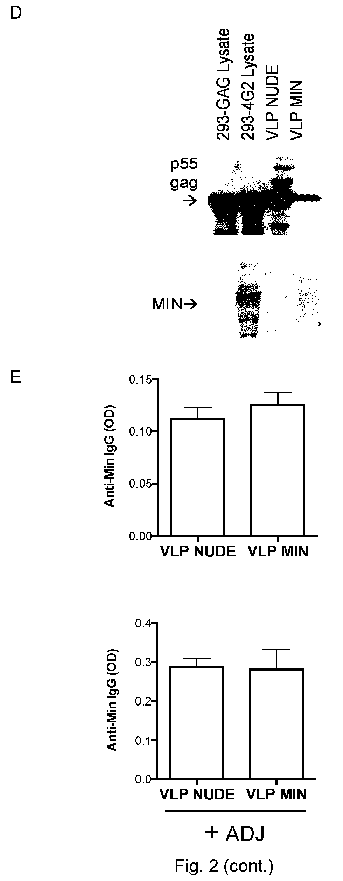

**Figure 2****. Characterization and immunogenicity of soluble MIN and MIN-VLPs obtained by cotransfection. Panel A.** The soluble MIN protein was expressed in *E. Coli* and purified to homogeneity by Ni²⁺ affinity and gel filtration chromatography. The final preparation contained a single 17 kDa band. Molecular weight markers are shown on the left. **Panel B.** The immunogenicity of soluble MIN preparations was tested in C57b16 mice. ELISA data against the MIN protein for sera (1/100 dilution) from control animals or animals immunized with MIN (50 µg/dose) with or without aluminum phosphate are shown. **Panel** C. HEK-293 cells stably transfected to express Env glycoprotein (NL4.3 isolate, clone K) (right), GAG (clone G1F2), MIN (clone, 4.2E6) or both Gag and MIN (left, clone 4G2A) were stained for cell surface expression of MPER epitopes and for intracellular expression of CA-p24 protein (Gag). Untransfected HEK-293 cells were included as negative control. Flow cytometry analysis is shown. **Panel D.** Cell pellets and supernatants from 293 G1F2 (expressing Gag) and 293 4G 2A cells (coexpressing both Gag and MIN) were analyzed by WB using an anti p24 antibody (upper) and the anti MPER antibody 2F5 (lower). **Panel E.** In parallel, the immunogenicity of MIN-VLP preparations was tested in C57b16 mice. ELISA data against the MIN protein for sera (1/100 dilution) from control animals or animals immunized with MINVLPs (50 µg total protein/dose) with or without aluminum phosphate are shown.

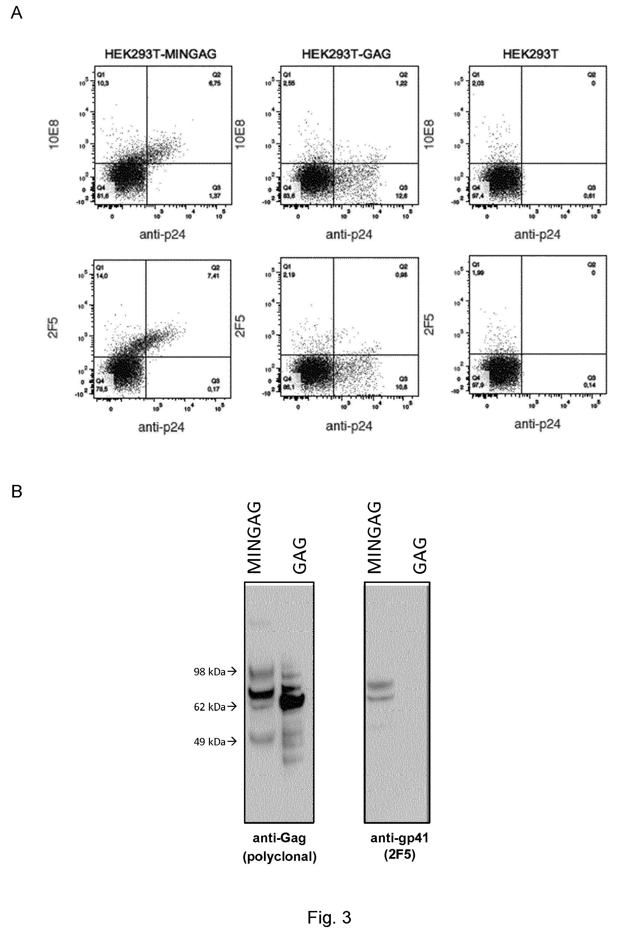

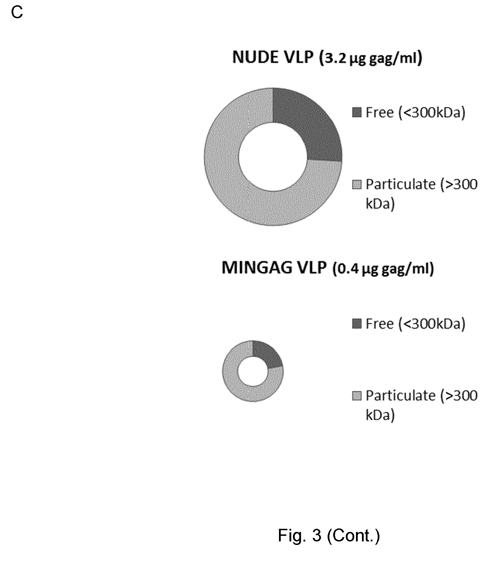

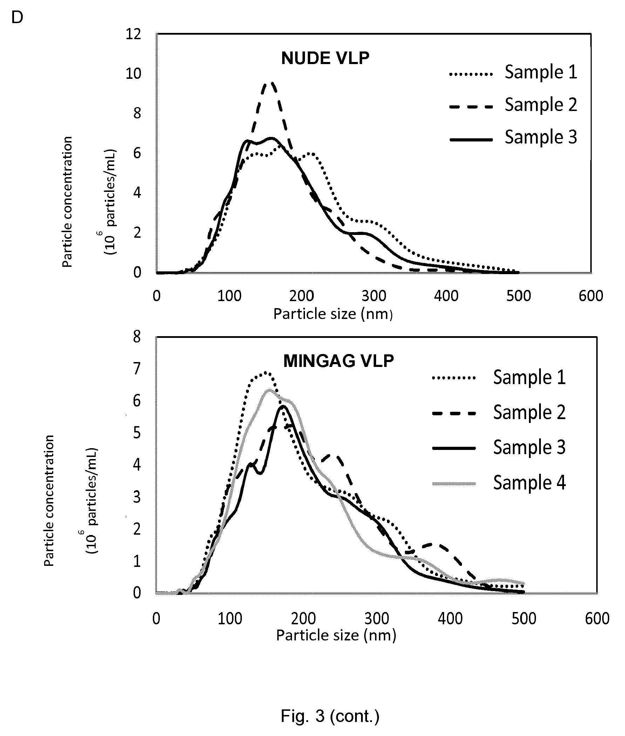

**Figure 3****. Characterization of MINGAG VLPs. Panel A.** 293T cells were transfected with the plasmid pcDNA3.1/MINGAG-3.7 coding for the MINGAG fusion protein, the plasmid pcDNA3.1 GAG coding for full length gag or with an empty pcDNA3.1 plasmid. After 24 hours, cells were cell surface stained with anti MPER antibodies 2F5 (top) or 10E8 (bottom) and intracellularly stained with the anti p24 antibody Kc57. Coexpression of both Gag and MPER antigens is clearly observed in both cases. **Panel B,** Supernatants from transfected cells were collected and assayed by Western Blot with a polyclonal anti gag antibody (left) and anti MPER antibody 2F5 (right)., Molecular weight markers are shown for reference). **Panel C.** Supernatants were also assayed for total p24 content before and after filtration through a 300 kDa molecular cutoff device. Values represent total p24 content (relative to diameter of the graph) and the fraction of particulate (VLP associated, light grey) and filterable (free, dark grey) material is shown. **Panel D.** Supernatants were assayed for particle diameter using Nanosight. Preparations of NUDE VLPs (top) or MINGAG VLPs (bottom) were assayed in triplicate or quadruplicate, respectively, with similar results (Panel D). Each line corresponds to individual determinations.

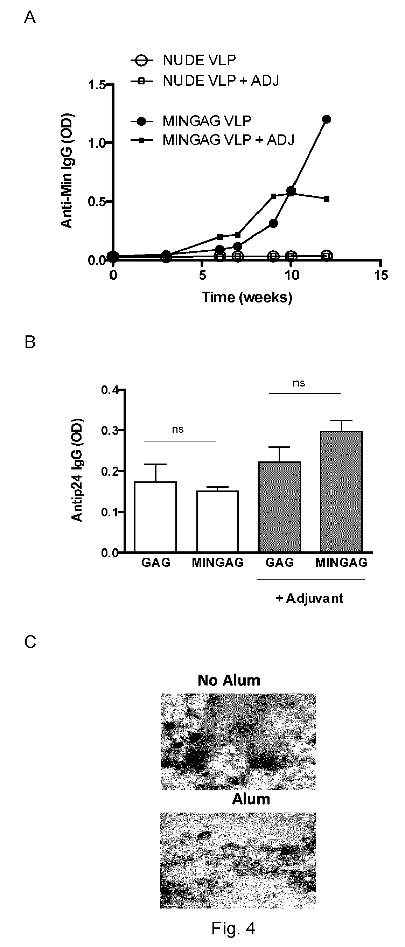

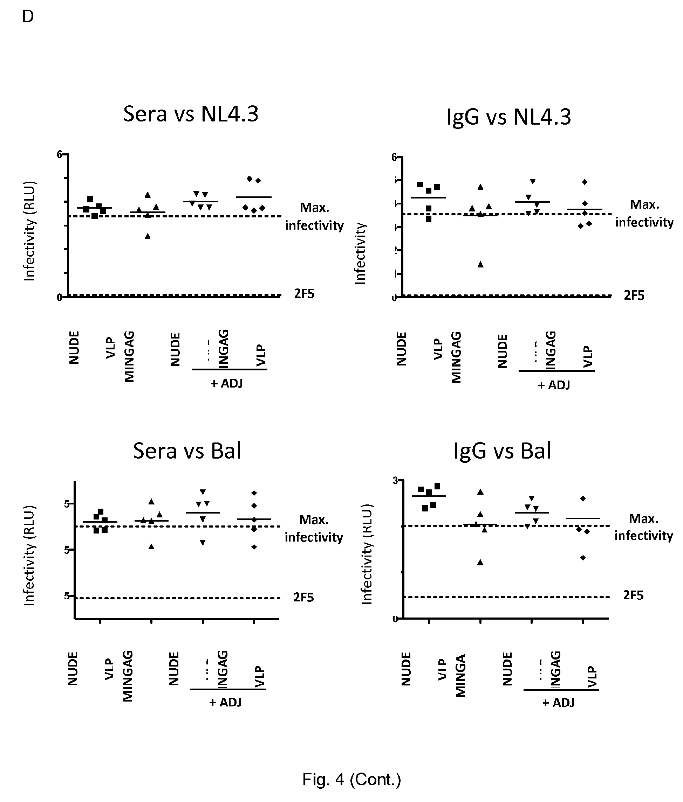

**Figure 4****. MINGAG VLP immunogenicity in mice. Panel A.** Time course of anti MIN IgG in mice immunized with NUDE VLPs or MINGAG VLPs with or without the adjuvant aluminum phosphate (+ADJ) . **Panel B.** Magnitude of anti-Gag IgG after sacrifice in mice immunized with NUDE VLPs or MINGAG VLPs with or without the adjuvant aluminum phosphate. **Panel C.** Electron microscopy analysis of untreated (top) and aluminum phosphate treated (bottom) VLP used for immunization. **Panel D.** Neutralizing activity of sera (1/100 dilution) and purified IgG (100 ug/mL) from sacrificed animals was assayed in TZM-bl cells against the indicated viruses. NL43 and BaL infectivity in the presence of individual animal sera or IgG is shown.

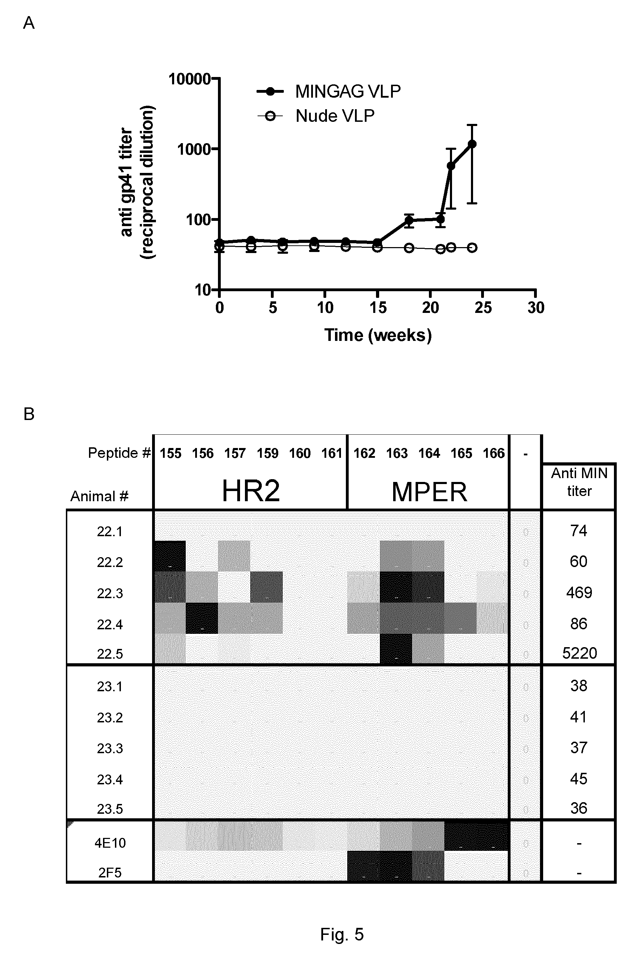

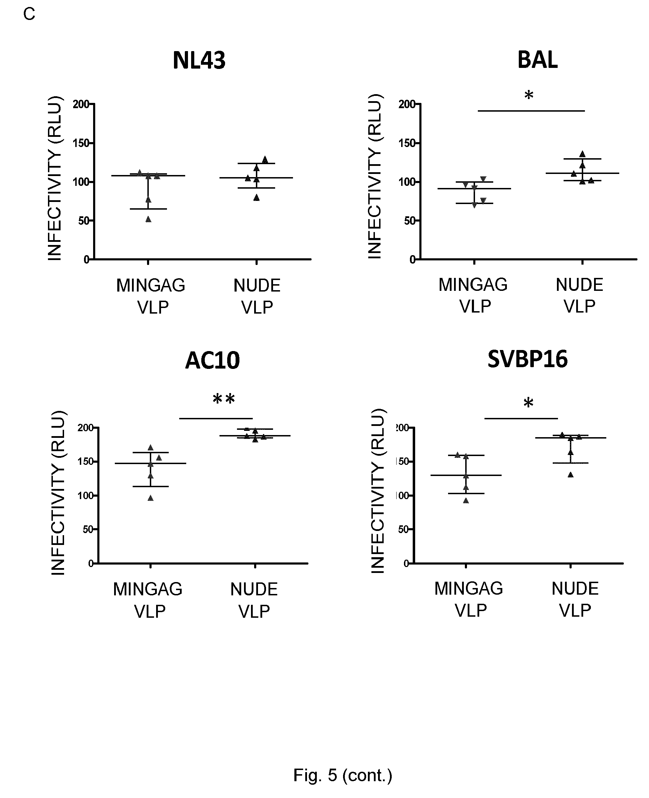

**Figure 5****. Peptide/VLP immunization in mice. Panel A.** Time course of anti MIN IgG in mice immunized with four doses of KLH-MPER and four doses of MINGAG VLPs or with control KLH and NUDE VLPs. **Panel B.** Mapping of anti-MIN IgG responses in immunized mice. Data show reactivity of serum obtained from individual animals against a set of 15mers overlapping peptides that cover the HR2 and MPER sequences of gp41 as described in methods. **Panel C.** Neutralizing activity of sera from sacrificed animals (1/100 dilution) was assayed in TZM-bl cells against the indicated viruses. Viral infectivity in the presence of individual animal sera is shown. Asterisks denote statistical significance.

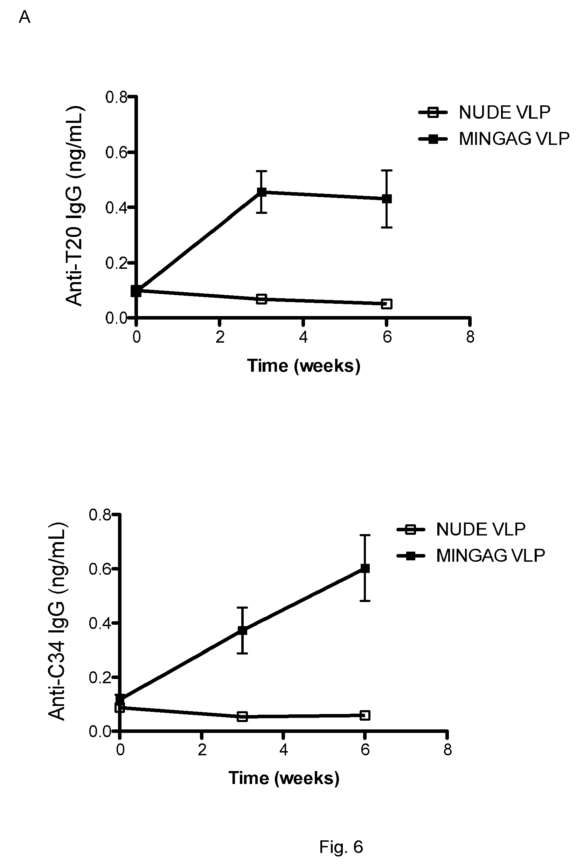

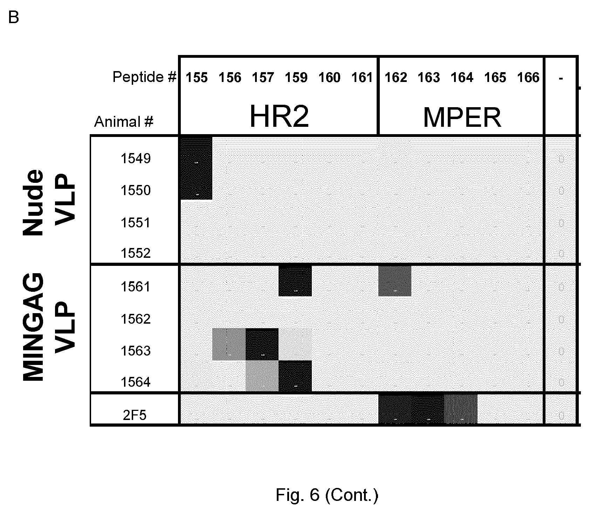

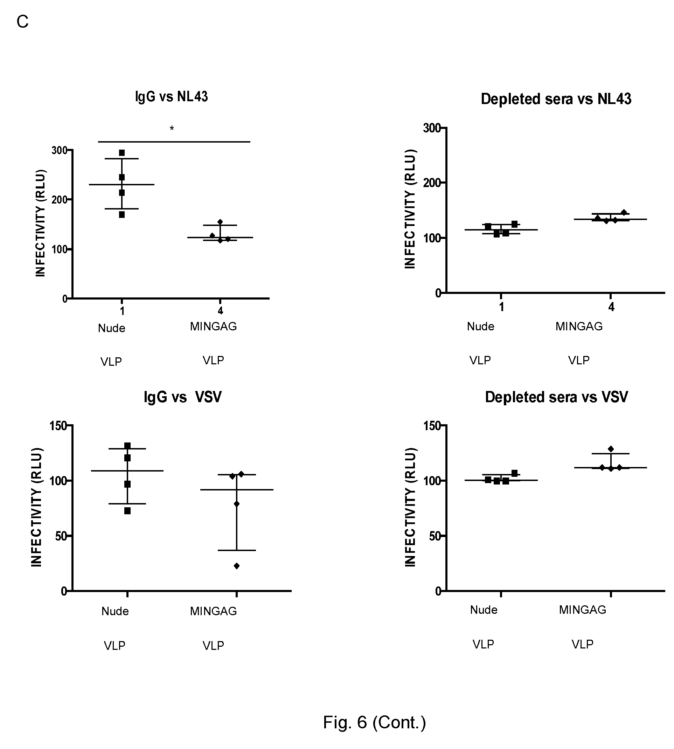

**Figure 6****. MINGAG VLP immunization in rabbits. Panel A.** Time course of anti T-20 and anti-C34 IgG in rabbits immunized with two doses of or NUDE VLPs MINGAG VLPs. **Panel B.** Mapping of anti-MIN IgG responses in immunized rabbits. Data show reactivity of serum samples obtained from immunized animals against a set of 15mers overlapping peptides that cover the HR2 and MPER sequences of gp41 as described in methods. **Panel C.** Neutralizing activity of purified IgG (100 µg/ml) and IgG depleted sera from sacrificed animals were assayed in TZM-bl cells against the indicated viruses. Viral infectivity in the presence of individual animal IgG is shown. Asterisks denote statistical significance.

### DEPOSIT OF MICROORGANISMS

The plasmid pcDNA3.1/MINGAG-3.7 was deposited on July 25, 2012 under access number DSM26214 at the DSMZ - Deutsche Sammlung von Mikroorganismen und Zellkulturen, Inhoffenstraße 7 B, D-38124 Braunschweig, Federal Republic of Germany.

### DETAILED DESCRIPTION OF THE INVENTION

The inventors have observed that one of the main limitations of the generation of neutralizing antibodies is the poor incorporation of antigens of interest into VLP *in vitro.* To overcome this limitation, the inventors engineered novel VLPs using gp41-based proteins (including the transmembrane domain) fused to gag. This design allowed for membrane localization of these proteins, maintained the VLP self-packing ability of Gag and showed increased gp41 surface density. The results clearly show increased immunogenicity of these newly engineered VLPs.

### Definitions

The term "fusion protein" as used herein, relates to proteins generated by gene technology which consist of two or more polypeptides derived from different proteins. A fusion protein may be obtained by conventional means (e.g. by means of gene expression of the nucleotide sequence encoding for said fusion protein in a suitable cell)

As used herein, "HIV" is meant to include HIV-1 and HIV-2. "HIV- 1" means the human immunodeficiency virus type-1. HIV-1 includes but is not limited to extracellular virus particles and the forms of HIV-1 associated with HIV-1 infected cells. "HIV-2" means the human immunodeficiency virus type-2. HIV-2 includes but is not limited to extracellular virus particles and the forms of HIV-2 associated with HIV-2 infected cells

"HIV-1_{JR-FL}" is a strain that was originally isolated from the brain tissue of an AIDS patient taken at autopsy and co-cultured with lectin-activated normal human PBMCs.

The term "gag polypeptide", as used herein relates to a primary protein product of the gag gene of HIV, which provides the basic physical infrastructure of the virus, and which is processed by viral protease during maturation to MA (matrix protein, p17); CA (capsid protein, p24); SP1 (spacer peptide 1, p2); NC (nucleocapsid protein, p7); SP2 (spacer peptide 2, p1) and P6 protein.

"A functionally equivalent variant", as used herein, relates to any sequence having additions, substitutions, deletions or combinations thereof in its amino acid sequence and/or which has been chemically modified with respect to said sequence and retains substantially equivalent ability to induce VLP formation in the case of a gag polypeptide variant or retains the ability to be embedded into the plasma membrane in the case of a functionally equivalent variant of the HIV gp41 polypeptide.

"Transmembrane domain", TM domain, as used herein relates to an amino acid sequence of approximately 25 hydrophobic residues with an occasional polar residue of integral proteins that pass across membrane.

As used herein, the term "transmembrane domain of the HIV gp41 polypeptide" refers to the region of the gp41 polypeptide that is embedded into the plasma membrane once gp41 has acquired its native topology.

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

The term "immunogenic polypeptide" as used herein, relates to a polypeptide of a protein from a pathogenic organism, tumor marker or allergen that, when administered to a mammal, are capable of inducing a protective immune response to the organism in the mammal.

As it is used in this description, the term "allergen" relates to a peptide or protein to which a subject is sensitive and causes an immune reaction, for example, allergen extracts of pollens, allergen extracts of insects, allergen extracts of food or food products, components present in saliva, insect claws or stings which induce a sensitivity reaction in a subject, components present in plants which induce a sensitivity reaction in a subject, etc.

The term "epitope" as used herein, refers to that portion of a given immunogenic substance that is the target of, i.e., is bound by, an antibody or cell-surface receptor of a host immune system that has mounted an immune response to the given immunogenic substance as determined by any method known in the art. Further, an epitope may be defined as a portion of an immunogenic substance that is recognized by a humoral or cellular, response, in particular an antibody response or a T-cell response in an animal, as determined by any method available in the art. An epitope can be a portion of any immunogenic substance, such as a protein, polynucleotide, polysaccharide, an organic or inorganic chemical, or any combination thereof. The term "epitope" may also be used interchangeably with "antigenic determinant" or "antigenic determinant site".

"Env" as used herein, relates to a viral glycoprotein that serves to form the viral envelope. The *env* gene codes for the gp160 glycoprotein which forms a homotrimer, and is cleaved into gp120 and gp41 by the host cell protease, furin. The full sequence of Env protein in isolate HXB2 has accession number P04578 in the UniProt database (6 July 2016)

The term "gp41", as used herein, refers to human immunodeficiency virus-1 envelope glycoprotein gp41. Gp41 is a subunit, which forms the Env glycoprotein of HIV-1 together with gp120. Env is a trimer composed of three external subunits (gp120) and three transmembrane subunits (gp41). The nucleic acid and amino acid sequences of a large number of HIV gp-41 are readily available to the public. See HIV Sequence Database, http://www.hiv.lanl.gov/content/sequence/HIV/mainpage.html, August 20010; Los Alamos HIV Databases and Compendia, http://www.hiv.lanl.gov/content/sequence/HIV/refer.html, August 2010.

As used herein, the term "heptad repeat 2" or HR2, refers to but is not limited to, a heptad repeat region that is located at the carboxy terminus of wild-type gp41. A heptad repeat is a motif in which a hydrophobic amino acid is repeated every seven residues; such motifs are designated a through *g. See* Lupas A, Trends Biochem. Sci. 1996; 21:375-382. Heptad repeats which contain hydrophobic or neutral residues at the a and d positions can form alpha helices and are able to interact with other heptad repeats by forming coiled *coils. See* Chambers P, et al., J. Gen. Virol. 1990; 71:3075-3080; Lupas A, *supra*.

The term "immunologically functional equivalent" as used herein refers to a variant of a polypeptide that retains substantially equivalent ability to induce an immune response in a subject as the reference polypeptide. The term "immunologically functional equivalent" is well understood in the art and is further defined in detail herein. Immunologically functional equivalents may increase the antigenicity of a polypeptide, maintain the same level of antigenicity of the reference polypeptide, or decrease the antigenicity of a polypeptide only slightly so that it maintains its usefulness as an antigen in an immunogenic composition.

"A product result from the processing of the Env protein" relates to any of the proteins produced by the processing of the gp160 protein.

The term "substantially equivalent", as used herein, refers to variants which are able to generate an immune response which is differs from the immune response generated with the native region by no more than 5 %, no more than 10 %, no more than 15 %, no more than 20 %, no more than 25 %, no more than 30 %, no more than 35 %, no more than least 40 %, no more than 45 %, no more than 50 %, no more than 55 %, no more than 60 %, no more than 65 %, no more than 70 %, no more than 75 %, no more than 80 %, no more than 85 %, no more than 90 % or no more than 95 %.

As used herein, the terms "membrane-proximal external region" and "MPER" refer, but are not limited to, a highly conserved region of the gp41 ectodomain adjacent to the viral membrane.

The phrase "stringent hybridization conditions" refers to conditions under which a probe will hybridize to its target subsequence, typically in a complex mixture of nucleic acid, but to no other sequences.

The term "linker region" or "linker", as used herein, refers to any heterologous polypeptide of at least 1, 2, 3, 4 or 5 or more amino acids in length, which when inserted between a first and second region yields a functional linkage joining both regions and wherein each region is capable of preserving its functional and immunological properties.

A "nucleic acid," "polynucleotide," or "nucleic acid molecule" is a polymeric compound comprised of covalently linked subunits called nucleotides. Nucleic acid includes ribonucleic acid (RNA) and deoxyribonucleic acid (DNA), both of which may be single-stranded or double-stranded. DNA includes cDNA, genomic DNA, synthetic DNA, and semi-synthetic DNA.

By the term "express", "expresses" or "expression" of a nucleic acid molecule it is meant that the sequence is transcribed, and optionally, translated. Typically, transcription and translation of a coding sequence will result in production of the immunogenic polypeptide according to the invention.

The term "codon optimised" as referred to herein relates to the alteration of codons in nucleic acid molecules to reflect the typical codon usage of the host organism, in the present case humans, without altering the polypeptide encoded by the DNA, to improve expression.

As used herein, the term "host cell" is intended to refer to a cell into which a nucleic acid of the invention, such as a recombinant expression vector of the invention, has been introduced.

The terms "host cell" and "recombinant host cell" are used interchangeably herein. It should be understood that such terms refer not only to the particular subject cell but to the progeny, or potential progeny, of such a cell. Because certain modifications may occur in succeeding generations due to either mutation or environmental influences, such progeny may not, in fact, be identical to the parent cell, but can be still included within the scope of the term as used herein.

As used herein, the term "recombinant bacterium" refers to any bacterium that has been modified by the introduction of heterologous DNA. "Wild-type" or "control" bacterium include bacterium as found in its natural state without genetic manipulation or that are substantially identical to the recombinant bacterium, but do not express one or more of the proteins encoded by the heterologous DNA (e.g. contains a plasmid without the coding sequence of the heterologous polypeptide of interest). The term is intended to include progeny of the bacterium originally modified by the introduction of heterologous DNA

The term "virus-like particle", also referred to as "VLP", relates to non-infectious particles resembling viruses that do not contain any viral genetic material. VLPs are the result of the expression of viral structural proteins, such as capsid proteins, and their self-assembly

The term "immunogenic composition", as used herein, refers to a composition that elicits an immune response in a subject that produces antibodies or cell-mediated immune responses against a specific immunogen.

The term "vaccine composition" refers to an immunogenic composition for *in vivo* administration to a host, which may be a primate, particularly a human host, to confer protection against disease, particularly a viral disease.

A "pharmaceutically acceptable carrier," "pharmaceutically acceptable diluent," or "pharmaceutically acceptable excipient", or "pharmaceutically acceptable vehicle," used interchangeably herein, refer to a non-toxic solid, semisolid or liquid filler, diluent, encapsulating material or formulation auxiliary of any conventional type.

Thus, "treatment," "treating," and the like, as used herein, refer to obtaining a desired pharmacologic and/or physiologic effect, covering any treatment of a pathological condition or disorder in a mammal, including a human. The effect may be prophylactic in terms of completely or partially preventing a disorder or symptom thereof and/or may be therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or at least symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter, such as inflammation, pain, and/or immune deficiency.

The terms "prevent," "preventing," and "prevention", as used herein, refer to a decrease in the occurrence of pathological cells in an animal. The prevention may be complete (e.g. the total absence of pathological cells in a subject). The prevention may also be partial, such that for example the occurrence of pathological cells in a subject is less than that which would have occurred without the present invention. Prevention also refers to reduced susceptibility to a clinical condition.

The term "subject" or "patient" is meant any subject, particularly a mammalian subject, for whom therapy is desired. Mammalian subjects include humans, domestic animals, farm animals, and zoo, sports, or pet animals such as dogs, cats, guinea pigs, rabbits, rats, mice, horses, cattle, cows, and so on.

The term "infectious disease", as used herein, relates to diseases caused by pathogens such as viruses, bacteria, fungi, protozoa, and parasites.

The term "neutralizing antibody" is any antibody or antigen-binding fragment thereof that binds to a pathogen and interferes with the ability of the pathogen to infect a cell and/or cause disease in a subject.

"bNAb" or broadly neutralizing antibody, as used herein is an antibody able to block the infectivity of a large proportion of HIV isolates from different clades.

"KLH", as used herein, relates to a large, multisubunit, oxygen-carrying, metalloprotein that is found in the hemolymph of the giant keyhole limpet, Megathura crenulata, a species of keyhole limpet that lives off the coast of California, from Monterey Bay to Isla Asuncion off Baja California.

### Fusion protein of the invention

In a first aspect, the invention relates to a fusion protein comprising a HIV gag polypeptide or a functionally equivalent variant thereof and an immunogenic polypeptide, wherein the immunogenic polypeptide is located N-terminal to the HIV gag polypeptide or to the functionally equivalent variant thereof

HIV is meant to include HIV-1 and HIV-2. The HIV-1 virus may represent any of the known major subtypes (Classes A, B, C, D E, F, G and H) or outlying subtype (Group O) including laboratory strains, primary isolates and circulating recombinant forms. The human immunodeficiency virus includes but is not limited to the JR-FL strain. Other commonly used HIV-1 strains such as HIV-1_{DH123}, HIV-1_{Gun-1}, HIV-1₈₉.₆, and HIV-1_{HXB2}.

In a preferred embodiment, the gag polypeptide or the functionally equivalent variant thereof is from HIV-1_{HXB2}.

The full sequence of gag polypeptide from HXB2 has accession number P04585-1 in the UniProt database (6 July 2016) and it is shown in SEQ ID NO: 1.

Preferably, the functional equivalent variants of the gag polypeptide show the aforementioned viral VLP formation activity at least by 60%, preferably by 70%, advantageously by 80%, more preferably by 90%, more preferably by 95%, even more preferably by 97% and even more preferably by 98%, advantageously by 99%. The skilled person knows several techniques to detect VLP formation of a functional variant of a HIV gag polypeptide.

Accordingly, functionally equivalent variant of a gag polypeptide show a degree of identity with respect to the amino acid sequence shown of the corresponding native gag polypeptide of at least 50%, at least 55%, at least 60%, at least 65%, at least 70%, at least 75%, at least 80%, at least 85%, at least 90%, at least 95%, at least 96%, at least 97%, at least 98% or at least 99% provided the VLP formation ability of the protein is maintained. Preferably, the identity exists over a region that is at least about 25 amino acids or nucleotides in length, or more preferably over a region that is 50-100 amino acids or nucleotides in length. An indication that two nucleic acid sequences or polypeptides are substantially identical is that the polypeptide encoded by the first nucleic acid is immunologically cross reactive with the antibodies raised against the polypeptide encoded by the second nucleic acid, as described below. Thus, a polypeptide is typically substantially identical to a second polypeptide, for example, where the two peptides differ only by conservative substitutions. The degree of identity between two peptides can be determined using computer algorithms and methods, which are widely known by the persons skilled in the art. The identity between two amino acid sequences of two peptides is preferably determined using the BLASTP algorithm. *See* Altschul, S. *et al.,* "BLAST Manual", (NCBI NLM NIH Bethesda, Md. 20894, Altschul, S., et al., J., 1990, Mol. Biol. 215:403-410). In a preferred embodiment, the degree of identity of the functionally equivalent variant with respect to gag is determined along the complete sequence of the variant, of gag or both.

In a preferred embodiment, the functionally equivalent variant of HIV gag lacks the myristoylation sequence.

Myristoylation is a lipidation modification where a myristoyl group derived from myristic acid, is covalently attached by an amide bond to the alpha-amino group of an N-terminal glycine residue. In the HIV gag polypeptide, the glycine susceptible to be myristoylated is the glycine in position 2 of SEQ ID NO: 1. The first methionine is removed by the host cell machinery.

In a preferred embodiment the functionally equivalent variant of HIV gag lacks the myristoylation sequence and in an even more preferred embodiment functionally equivalent variant of HIV gag has the sequence shown in SEQ ID NO: 2.

In another preferred embodiment, the fusion protein contains a transmembrane domain between the immunogenic polypeptide and the HIV gag polypeptide or a functionally equivalent variant.

Any single-pass transmembrane protein may be used in the present invention, by way of illustrative non-limitative examples the transmembrane domain of CD22 (SEQ ID NO: 18), CD36 (SEQ ID NO: 19) and CD44 (SEQ ID NO: 20)

Several assays known in the art may be used in order to determine whether a sequence forms a transmembrane domain.

In a preferred embodiment, the transmembrane domain is the transmembrane domain of the HIV gp41 polypeptide or a functionally equivalent variant thereof.

In the particular embodiment, the transmembrane region of the HIV gp41 polypeptide is the sequence shown in SEQ ID NO: 17.

The fusion protein according to the invention comprises the immunogenic polypeptide located N-terminal to the HIV gag polypeptide or the functionally equivalent variant.

The immunodominant polypeptide may comprise one or more epitopes. Generally, but not always, such immunodominant polypeptide or epitopes are highly immunogenic when tested according to methods that are known to those of skill in the art.

In a preferred embodiment, the immunogenic polypeptide is a polypeptide capable of (suitable or designed for) inducing an immune response against an infectious disease, such as an infectious disease in animals caused by pathogenic microorganisms of animals, including humans, for example, virus, bacteria, fungi and infectious parasites, relevant in human or animal health.

The proteins or peptides capable of inducing an immune response can be recombinant proteins or peptides, identical or similar to the natural antigens of a specific microorganism.

Non-limiting illustrative examples of infectious virus include virus of the families: Arteriviridae, Retroviridae, Picornaviridae, Calciviridae, Togaviridae, Flaviridae, Coronoviridae, Rhabdoviradae, Filoviridae, Paramyxoviridae, Orthomyxoviridae, Bungaviridae, Arenaviridae, Reoviridae, Birnaviridae, Hepadnaviridae, Parvoviridae (parvovirus), Papovaviridae, Adenoviridae, Herpesviridae, Poxviridae, Iridoviridae, etc. Examples of antigens which can be used according to the present invention include but are not limited to HIV antigens, gp 120 antigen, hepatitis B surface antigen, rotavirus antigens such as VP4 and VP7, influenza virus antigens such as hemagglutinin or nucleoprotein, thymidine kinase herpes simplex antigen, etc.

Non-limiting illustrative examples of bacteria include both Gram positive bacteria, e.g., *Pasteurella sp., Staphylococcus sp., Streptococcus sp.,* etc., and Gram negative bacteria, e.g., *Escherichia coli, Pseudomonas sp., Salmonella sp., etc. Specific examples of infectious bacteria include: Helicobacter pylori, Borelia burgdorferi, Legionella pneumoplailia, Mycobacteria sp.* (e.g., *M. tuberculosis, M. avium, M. intracellulare, M. kansaii, M. gordonae), Staphylococcus aureus, Neisseria gonorrhoeae, Neisseria meningitidis, Listeria monocytogenes, Streptococcus pyogefaes (Streptococcus Group A), Streptococcus agalactiae (Streptococcus Group B), Streptococcus (viridans group), Streptococcus faecalis, Streptococcus bovis, Streptococcus (anaerobic species), Streptococcus pneumoniae, Campylobacter sp., Enterococcus sp., Haemophilus influenzae, Bacillus aratracis, Corynebacterium diphtheriae, Corynebacterium sp., Erysipelothrix rhusiopathiae, Clostridium perfringers, Clostridium tetani, Enterobacter aerogenes, Klebsiella pneumoniae, Pasturella multocida, Bacteroides sp., Fusobacterium nucleatum, Streptobacillus moniliformis, Treponemapallidium, Treponema pertenue, Leptospira, Rickettsia, Actinornyces israelli, Chlamydia,* etc.

Non-limiting illustrative examples of infectious fungi include *Cryptococcus neoformans, Histoplasma capsulatum, Coccidioides immitis, Blastomyces dermatitidis, Chlamydia trachomatis and Candida albicans.*

By way of non-limiting illustration, protozoa are included among the infectious parasites, such as *Plasmodium sp., protozoa causing malaria, e.g. P. falciparum, P. malariae, P. ovale, P. vivax, etc., Leishmania sp., protozoa causing leishmaniasis, e.g., L. major, L. donovani, L. infantum, L. braziliensis, L. panamensis, L. mexicana, etc., Toxoplasma gondii, Schistosoma sp., etc.,* as well as parasitic nematodes, such as *Dirofilaria immitis,* protozoa causing the Chagas disease, e.g. *Tripanosoma cruzi, protozoa causing African trypanosomiasis, e.g. Tripanosoma brucei, etc. Examples of antigens for these parasites include Plasmodium spp. circumsporozoite antigen; Plasmodium spp. merozoite surface antigen; Leishmania spp. gp63,* etc.

In another preferred embodiment, the immunogenic polypeptide is a polypeptide associated with tumors or cancers ("tumor markers") capable of (suitable or designed for) inducing an immune response against a tumor or cancer cell, therefore the fusion protein polypeptide of interest can be used in the treatment of cancers by means of the stimulation of an antigen-specific immune response against a tumor antigen.

Non-limiting illustrative examples of cancers which could be potentially treated according to the teachings of the present invention include bile duct cancer, brain cancer, breast cancer, cervical cancer, choriocarcinoma, colon cancer, endometrial cancer, esophageal cancer, stomach cancer, intraepithelial neoplasias, lymphomas, liver cancer, lung cancer (e.g., small and non-small cell lung cancer), melanoma, neuroblastomas, mouth cancer, ovarian cancer, pancreatic cancer, prostate cancer, rectal cancer, sarcomas, skin cancer, testicular cancer, thyroid cancer and renal cancer, as well as other carcinomas and sarcomas.

A person skilled in the art can select tumor said antigens and antigenic determinants being included within the scope of the present invention. Representative examples of said antigens or antigenic determinants include: Her2 (breast cancer); GD2 (neuroblastoma); EGF-R (malignant glioblastoma); CEA (medullary thyroid cancer); CD52 (leukemia); human melanoma gp100 protein; human melanoma melan-A/MART-1 protein; tyrosinase; NA17-A nt protein; MAGE-3 protein; p53 protein; HPV16E7 protein; and antigenic fragments of said peptides or proteins.

In another preferred embodiment the immunogenic polypeptide is a polypeptide capable of (suitable or designed for) inducing an immune response against an allergen.

Non-limiting illustrative examples of allergens include protein extracts of pollens, e.g., of *Lolium perenne, Poa pratense, Phleum pratense, Cynodon dactylon, Festuca pratensis, Dactylis glomerata*, *Secale cereale, Hordeum vulgare*, *Avena sativa, Triticum sativa, Artemisia vulgaris*, *Chenopodium album*, *Plantago lanceolata, Taraxacum vulgare*, *Parietaria judaica, Salsola kali, Urtica dioica, Olea europea, Platanus sp., Cupressus sp.,* etc.; protein extracts of insects, e.g., of *Dermatophagoides pteronyssinus, Dermatophagoides farinae*, *Acari such as Acarus siro, Blomia tropicalis, Euroglyphus maynei*, *Glyciphagus domesticus*, *Lepidoglyphus destructor, Tyrophagus putrescentiae*, etc.; protein extracts of fungi or of animal dander, e.g., *Penicillium sp., Alternaria alternata, Cladosporium herbarum*, dog dander, cat dander, horse dander, etc.; protein extracts of food or food products, etc.

In a preferred embodiment, the immunogenic polypeptide is from a protein of a virus. In a more preferred embodiment, the virus is HIV.

In a preferred embodiment, the immunogenic polypeptide does not contain an epitope from the HIV gag polypeptide.

In another preferred embodiment, the immunogenic polypeptide is from an Env protein of the HIV virus or a product results from the processing of the Env protein.

In a preferred embodiment, the sequence of the Env protein of HIV is the sequence shown in SEQ ID NO: 21

In a preferred embodiment the product resulting from the processing of the Env protein, is gp41 or a functionally equivalent variant.

Preferably, the functional equivalent variants of the gp41 polypeptide show the aforementioned viral VLP formation activity at least by 60%, preferably by 70%, advantageously by 80%, more preferably by 90%, more preferably by 95%, even more preferably by 97% and even more preferably by 98%, advantageously by 99%. The skilled person knows several techniques to detect VLP formation of a functional variant of a HIV gag polypeptide.

In a preferred embodiment, the gp41 variant sequence is shown in SEQ ID NO: 5 and comprises the gp41 HR2 region and the MPER region.

In another preferred embodiment, the immunogenic polypeptide has less than 90 amino acids, preferably less than 80 amino acids, more preferably less than 70 aminoacids and even more preferably less than 60 amino acids.

In another preferred embodiment, the immunogenic polypeptide comprises from the N to C terminus,
a) the heptad repeat 2 of the HIV gp41 polypeptide or an immunologically equivalent variant thereof, and
b) the membrane proximal external region of the HIV gp41 polypeptide or an immunologically equivalent variant thereof.

The gp41 HR1 and HR2 sequences are well known in the art. In the particular case the HR2 region shows the sequence depicted in SEQ ID NO:22

In the particular case of the HXB2 Env protein, the MPER region shows the sequence depicted in SEQ ID NO:23.

As modifications and changes may be made in the structure of the regions forming the gp41 variants according to the invention and still obtain molecules having like or otherwise desirable characteristics, such immunologically functional equivalents are also encompassed within the scope of the present invention.

For the purposes of the present invention, a polypeptide that is useful as an antigen in an immunogenic composition (i.e. has sufficient "immunogenic activity") can be identified by any method commonly known in the art for measuring the ability of a given polypeptide to trigger the generation of antibodies specific for said polypeptide when administered to a host organism. The ability of a given variant of the polypeptide of the invention to be an immunologically equivalent variant may be determined using standard neutralization assays, wherein the suspected variant is inoculated into a test animal and the resulting antibodies are tested for their ability to neutralize the infection of susceptible cells by HIV strains.

Immunologically functional equivalents of the immunogenic polypeptide according to the invention can be obtained by modifying the polypeptide. Said modifications can be, without limitation, amino acid changes, deletions, truncations, polypeptide fragments, fusions to other polypeptides, insertions, or any combination thereof Accordingly, immunologically functional equivalents of the immunogenic polypeptide show a degree of identity with respect to the amino acid sequence shown of the corresponding region in the native immunogenic polypeptide of at least 50%, at least 55%, at least 60%, at least 65%, at least 70%, at least 75%, at least 80%, at least 85%, at least 90%, at least 95%, at least 96%, at least 97%, at least 98% or at least 99% provided the immunogenic activity of the protein is maintained. Preferably, the identity exists over a region that is at least about 25 amino acids or nucleotides in length, or more preferably over a region that is 50-100 amino acids or nucleotides in length. An indication that two nucleic acid sequences or polypeptides are substantially identical is that the polypeptide encoded by the first nucleic acid is immunologically cross reactive with the antibodies raised against the polypeptide encoded by the second nucleic acid, as described below. Thus, a polypeptide is typically substantially identical to a second polypeptide, for example, where the two peptides differ only by conservative substitutions. The degree of identity between two peptides can be determined using computer algorithms and methods, which are widely known by the persons skilled in the art. The identity between two amino acid sequences of two peptides is preferably determined using the BLASTP algorithm. *See* Altschul, S. *et al.,* "BLAST Manual", (NCBI NLM NIH Bethesda, Md. 20894, Altschul, S., et al., J., 1990, Mol. Biol. 215:403-410).

As an example of modifications contemplated to be within the scope of the present invention, certain amino acids may be substituted for other amino acids in a polypeptide structure without appreciable loss of interactive binding capacity of the structure such as, for example, the epitope of an antigen that is recognized and bound by an antibody. Since it is the interactive capacity and nature of a polypeptide that defines its biological (e.g. immunological) functional activity, certain amino acid sequence substitutions can be made in an amino acid sequence (or its underlying DNA coding sequence) and nevertheless obtain a polypeptide with comparable properties. Various changes may be made to the amino acid sequences of the antigens of the present invention without appreciable loss of immunogenic activity.

It is understood in the art that in order to make functionally equivalent amino acid substitutions, the hydropathic index of amino acids may be considered. The importance of the hydropathic amino acid index in conferring interactive biological function on a polypeptide is generally understood in the art. *See* Kyte J, Doolittle R, J. Mol. Biol. 1982; 15(1):105-132. It is known that certain amino acids may be substituted for other amino acids having a similar hydropathic index or score and still retain a similar biological activity. In making changes based upon the hydropathic index, the substitution of amino acids whose hydropathic indices are within plus or minus 2 is preferred, those which are within plus or minus 1 are particularly preferred, and those within plus or minus 0.5 are even more particularly preferred. Each amino acid has been assigned a hydropathic index on the basis of its hydrophobicity and charge characteristics; these are: isoleucine (+4.5), valine (+4.2), leucine (+3.8), phenylalanine (+2.8), cysteine/cystine (+2.5), methionine (+1.9), alanine (+1.8), glycine (-0.4), threonine (-0.7), serine (-0.8), tryptophan (-0.9), tyrosine (-1.3), proline (-1.6), histidine (-3.2), glutamate (-3.5), glutamine (-3.5), aspartate (-3.5), asparagine (-3.5), lysine (-3.9) and arginine (-4.5).

It is also understood in the art that the substitution of similar amino acids can be made effectively on the basis of hydrophilicity; particularly where the immunologically functional equivalent polypeptide thereby created is intended for use in immunological embodiments, as in certain embodiments of the present invention. The greatest local average hydrophilicity of a protein, as governed by the hydrophilicity of its adjacent amino acids, correlates with its immunogenicity and antigenicity. *See* Hopp T, US 4,554,101. In making changes based upon similar hydrophilicity values, the substitution of amino acids whose hydrophilicity values are within plus or minus 2 is preferred, those which are within plus or minus 1 are particularly preferred, and those within plus or minus 0.5 are even more particularly preferred. The following hydrophilicity values have been assigned to amino acid residues: arginine (+3.0), lysine (+3.0), aspartate (+3.0 plus or minus 1), glutamate (+3.0 plus or minus 1), serine (+0.3), asparagine (+0.2), glutamine (+0.2), glycine (0), threonine (-0.4), proline (-0.5 plus or minus 1), alanine (-0.5), histidine (-0.5), cysteine (-1.0), methionine (-1.3), valine (-1.5), leucine (-1.8), isoleucine (-1.8), tyrosine (-2.3), phenylalanine (-2.5) and tryptophan (-3.4).

It is well known in the art that where certain residues are shown to be particularly important to the immunological or structural properties of a protein or peptide, like for example, residues in binding regions or epitopes, such residues may not generally be exchanged. In this manner, functional equivalents are defined herein as those polypeptides, which maintain a substantial amount of their native immunological activity. In general, the shorter the length of the molecule, the fewer changes can be made to the molecule without affecting its function. Longer domains may have an intermediate number of changes. The full-length protein will have the most tolerance for a larger number of changes. However, it must be appreciated that certain molecules or domains that are highly dependent upon their structure may tolerate little or no modification.

Immunologically equivalent variants can also be obtained by the expression from nucleic acid sequences which are substantially identical to the molecule encoding the regions in the native immunogenic polypeptide or their complements hybridize to each other under stringent conditions, as described below, provided that the immunogenic activity of the polypeptide encoded by said nucleic acids is preserved.

Stringent conditions are sequence-dependent and will be different in different circumstances. Longer sequences hybridize specifically at higher temperatures. *See* Tijssen S, "Overview of principles of hybridization and the strategy of nucleic acid assays", Laboratory Techniques in Biochemistry and Molecular Biology (Elsevier Science Publishers B.V., Amsterdam, The Netherlands 1993). Generally, stringent conditions are selected to be about 5-10°C degrees lower than the thermal melting point (Tm) for the specific sequence at a defined ionic strength pH. The Tm is the temperature (under defined ionic strength, pH, and nucleic concentration) at which 50 percent of the probes complementary to the target hybridize to the target sequence at equilibrium (as the target sequences are present in excess, at Tm 50 percent of the probes are occupied at equilibrium). Stringent conditions will be those in which the salt concentration is less than about 1.0 M sodium ion, typically about 0.01 to 1.0 M sodium ion concentration (or other salts) at pH 7.0 to 8.3 and the temperature is at least about 30°C for short probes (e.g. 10 to 50 nucleotides) and at least about 60° C for long probes (e.g. greater than 50 nucleotides). Stringent conditions may also be achieved with the addition of destabilizing agents such as formamide. For high stringency hybridization, a positive signal is at least two times background, preferably 10 times background hybridization. Exemplary high stringency or stringent hybridization conditions include: 50 percent formamide, 5xSSC and 1 percent SDS incubated at 42°C or 5xSSC and 1 percent SDS incubated at 65°C, with a wash in 0.2xSSC and 0.1 percent SDS at 65°C.

A person skilled in the art will appreciate that the different components which form part of the fusion protein according to the present invention may be functionally linked directly or indirectly through a linker region.

Therefore, in a particular embodiment, the fusion protein further comprises a linker. IN a more preferred embodiment, the linker comprises glycine residues, more preferably glycine and serine residues, even more preferably the linker comprises three glycine residues and one serine residue. In a more preferably embodiment, the linker is selected from the sequence shown in SEQ ID NO: 24 and 25.

In another preferred embodiment, the fusion protein of the invention further comprise a tag which may be used for the detection or for the purification of the gp41 variant using reagents showing specific affinity towards said tags. Adequate detection/purification tags includes hexa-his (metal chelate moiety), hexa-hat GST (glutathione S-tranferase) glutathione affinity, calmodulin-binding peptide (CBP), streptag, cellulose binding domain, maltose binding protein, S-peptide tag, chitin binding tag, immuno-reactive epitopes, epitope tags, E2tag, HA epitope tag, Myc epitope, FLAG epitope, AU1 and AU5 epitopes, GIu-GIu epitope, KT3 epitope, IRS epitope, Btag epitope, protein kinase-C epitope, VSV epitope or any other tag as long as the tag does not affect the stability of the protein or the immunogenicity of the antigen attached thereto.

Moreover, different types of amino acid sequences may be added to the fusion protein to attain different objectives such as, for example, facilitating their handling or monitoring their expression. The addition of these peptide sequences to the fusion protein does not affect their immunogenic capabilities.

In a preferred embodiment the linker sequence is selected from the group consisting of SEQ ID NO: 26, SEQ ID NO: 27 and SEQ ID NO: 28. In another preferred embodiment, the components if the fusion protein comprises several linker sequences.

In another preferred embodiment, the fusion protein according to the invention has the sequence shown in SEQ ID NO: 29.

### Nucleic acids, vectors, host cells of the invention

In another aspect, the invention relates to a polynucleotide encoding a fusion protein according to the invention.

In a preferred embodiment, the polynucleotide of the invention has the sequence shown in SEQ ID NO: 9.

In another preferred embodiment, the sequence of the polynucleotide according to the invention is codon-optimised for expression in humans. In another preferred embodiment the codon-optimised sequence coding for the immunogenic polypeptide of the invention is shown in SEQ ID NO: 11 and the codon-optimised sequence coding for the HIV gag polypeptide of the invention has the sequence shown in SEQ ID NO: 13

A plethora of methods and software tools for codon optimisation are known to the skilled person. Codon-optimised nucleic acids for use according to the present invention can be prepared by replacing the codons of the nucleic acid encoding the immunogen by "humanized" codons, i.e., the codons are those that appear frequently in highly expressed human genes.

In a preferred embodiment, the polynucleotide of the invention encoding the fusion protein of the invention is codon-optimised for expression in humans has the sequence shown in SEQ ID NO: 15.

The polynucleotide of this invention can be operably linked to any promoter and/or enhancer capable of driving expression of the nucleic acid following introduction into a host cell. A promoter is an array of nucleic acid control sequences that directs transcription of a nucleic acid. A promoter includes necessary nucleic acid sequences (which can be) near the start site of transcription, such as in the case of a polymerase II type promoter (a TATA element). A promoter also can include distal enhancer or repressor elements which can be located as much as several thousand base pairs from the start site of transcription. Both constitutive and inducible promoters are included.

The polynucleotides of the invention can be incorporated into a vector, for the purposes of cloning, performing other laboratory manipulations, manufacturing recombinant peptides or gene delivery.

Therefore, in another aspect, the invention relates to a vector comprising a polynucleotide according to the invention.

A person skilled in the art will understand that there is no limitation as regards the type of vector that can be used. The vector can be a cloning vector, suitable for propagation and for obtaining polynucleotides, gene constructs or expression vectors incorporated to several heterologous organisms. Thus, suitable vectors according to the present invention include prokaryotic expression vectors (e.g. pUC18, pUC19, Bluescript and their derivatives), mp18, mp19, pBR322, pMB9, CoIE1, pCR1, RP4, phages and shuttle vectors (e.g. pSA3 and pAT28), yeast expression vectors (e.g. vectors of the type of 2 micron plasmids), integration plasmids, YEP vectors, centromeric plasmids and the like, insect cell expression vectors (e.g. the pAC series and pVL series vectors), plant expression vectors, such as vectors of expression in plants (e.g. pIBI, pEarleyGate, pAVA, pCAMBIA, pGSA, pGWB, pMDC, pMY, pORE series vectors), and eukaryotic expression vectors based on viral vectors (e.g. adenoviruses, viruses associated to adenoviruses as well as retroviruses and lentiviruses), as well as non-viral vectors such as pSilencer 4.1-CMV (Ambion, Applied Biosystems, Foster City, CA, USA), pcDNA3, pcDNA3.1/hyg pHCMV/Zeo, pCR3.1, pEFl/His, pIND/GS, pRc/HCMV2, pSV40/Zeo2, pTRACER-HCMV, pUB6/V5-His, pVAX1, pZeoSV2, pCI, pSVL and pKSV-10, pBPV-1, pML2d and pTDT1. In a preferred embodiment, the vector is pcDNA3.1.

In a particular embodiments, the vector is an expression vector that can include a regulatory element, such as the cytomegalovirus hCMV immediate early gene, the early promoter of SV40 adenovirus, the late promoter of SV40 adenovirus, the lac system, the TAC system, the TRC system, the major operator and promoter regions of phage, the control regions of fd coat protein, the promoter for 3-phosphoglycerate kinase, the promoters of acid phosphatase or the promoters of the yeast mating factors. Exemplary vectors include bacterial artificial chromosomes, cosmids, yeast artificial chromosomes, phage, plasmids, lipid vectors and viral vectors (e.g. retrovirus). Particularly preferred are expression vectors which express the peptide of the invention as part of a fusion protein. Suitable fusion protein expression vectors have been described. *See* Kayman, et al., US 5,643,756.

In another embodiment, the invention relates to a host cell which comprises the fusion protein of the invention, the polynucleotides encoding them or the vectors comprising the polynucleotides.

Cells suitable in the present invention include, without limitation, mammal, plant, insect, fungal and bacterial cells. Bacterial cells include, without being limited to, Gram-positive bacterial cells such as species of the *Bacillus, Streptomyces* and *Staphylococcus* genus and Gram-negative bacterial cells such as cells of the *Escherichia* and *Psendomonas* genus. Fungal cells preferably include cells of yeasts such as *Saccharomyces, Pichia pastoris* and *Hansenula polymorpha.* Insect cells include, without being limited to, Drosophila cells and Sf9 cells. Plant cells include, among others, cells of crop plants such as cereals, medicinal, ornamental or bulbous plants. Mammalian cells include, without limitation, Chinese hamster ovary (CHO) cells such as CHO-K1 (ATCC Accession No. CCL-61), DG44 (Chasin L, et al., 1986, Som. Cell Mol. Genet., 12:555-556; and Kolkekar A, et al., 1997, Biochemistry, 36:10901-10909), CHO-K1 Tet-On (Clontech, Mountain View, CA, USA), CHO designated ECACC 85050302 (CAMR, Salisbury, Wiltshire, UK), CHO clone 13 (GEIMG, Genova, IT), CHO clone B (GEIMG, Genova, IT), CHO-K1/SF designated ECACC 93061607 (CAMR, Salisbury, Wiltshire, UK), RR-CHOK1 designated ECACC 92052129 (CAMR, Salisbury, Wiltshire, UK), CHO cells negative for dihydrofolate reductase (CHO/-DHFR, Urlaub G, Chasin L, Proc. Natl. Acad. Sci. USA; 77:4216-4220), SV40-transformed monkey kidney CV1 cells (COS, COS-7, ATCC Accession No. CRL-1651); human embryonic kidney cells (293, 293T cells); baby hamster kidney cells (BHK, ATCC Accession No. CCL-IO); monkey kidney cells (CV1, ATCC Accession No. CCL-70); African Green Monkey kidney cells (VERO-76, ATCC Accession No. CRL-1587; VERO, ATCC Accession No. CCL-81); mouse Sertoli cells (TM4, Mather J, Biol. Reprod. 1980; 23:243-251); human cervical carcinoma cells (HELA, ATCC Accession No. CCL-2); dog kidney cells (MDCK, ATCC Accession No. CCL-34); human lung cells (W138, ATCC Accession No. CCL-75); human hepatoma cells (HEP-G2, HB 8065); mouse mammary tumor cells (MMT 060562, ATCC Accession No. CCL-51); buffalo rat liver cells (BRL 3A, ATCC Accession No. CRL- 1442); TRI cells (Mather J, Ann. NY Acad. Sci. 1982, 383:44-68); MCR 5 cells and FS4 cells. Preferably, the host cells utilized are HEK-293T cells.

In a preferred embodiment, the host cell of the invention is a recombinant bacterium.

In a preferred embodiment, the bacterium is a gram negative bacterial cell, and this term is intended to include all facultative Gram-negative cells of the family *Enterobacteriaceace* such as *Escherichia, Shigella, Citrobacter, Salmonella, Klebsiella, Enterobacter, Erwinia, Kluyvera, Serratia, Cedecea, Morganella, Hafhia, Edwardsiella, Providencia, Proteus and Yersinia.*

All the terms and embodiments previously described in relation to the fusion protein the invention are equally applicable to polynucleotide, vector or host cell of the invention.

### Method for preparing a VLP, and VLP of the invention

In another aspect, the invention relates to a method for preparing a VLP loaded with an immunogenic polypeptide comprising
(i) expressing in a cell a fusion protein according to the invention and
(ii) recovering the VLPs from the extracellular medium

The first step of the method of the invention comprises the transformation of a host cell with a vector comprising the polynucleotide of the invention. Said transformation can be carried out by conventional techniques that are well known to those of ordinary skill in the art. Where the host is prokaryotic, such as *E. coli,* competent cells which are capable of DNA uptake can be prepared from cells harvested after exponential growth phase and subsequently treated by the CaCl₂ method using procedures well known in the art. Alternatively, MgCl₂ or RbCl can be used. Transformation can also be performed after forming a protoplast of the host cell if desired, or by electroporation.

When the host is a eukaryote, such methods of transfection of DNA as calcium phosphate coprecipitates, conventional mechanical procedures such as microinjection, electroporation, insertion of a plasmid encased in liposomes, or virus vectors can be used. Eukaryotic cells can also be co-transformed with polynucleotide sequences encoding an immunogenic gp120 polypeptide, and a second foreign DNA molecule encoding a selectable phenotype, such as the herpes simplex thymidine kinase gene. Another method is to use a eukaryotic viral vector, such as simian virus 40 (SV40) or bovine papilloma virus, to transiently infect or transform eukaryotic cells and express the protein

The cell culture should be maintained under suitable conditions to allow the polynucleotide of the invention to be expressed.

The second step of the method of the invention comprises recovering the VLP from the extracellular medium, for example by centrifugation and filtration tthrough 0.45 µm filters.

In the case of the VLPs obtained using the fusion proteins of the invention, the VLPs are characterized in that the viral capsid is enclosed by a lipidic envelope, which is a lipid bilayer derived from the cell in which the VLP has been formed.

In another aspect, the invention relates to a virus like-particle comprising the fusion protein according to the invention or obtained using a method of the invention.

All the terms and embodiments previously described are equally applicable to the method for preparing a VLP of the invention and to the VLP of the invention.

### Immunogenic composition or vaccine composition

In another aspect, the invention relates to an immunogenic composition or a vaccine composition comprising an immunogenically amount of a fusion protein according to the invention, a polynucleotide according to the invention, a vector according to the invention, a host cell according to the invention or a virus like-particle according to the invention.

The immunogenic compositions or vaccine composition according to the invention may further comprise a pharmaceutically acceptable carrier.

A pharmaceutically acceptable carrier is essentially non-toxic to recipients at the dosages and concentrations employed, and is compatible with other ingredients of the formulation. For example, the carrier for a formulation containing polypeptides would not normally include oxidizing agents and other compounds that are known to be deleterious to polypeptides. Suitable carriers include, but are not limited to water, dextrose, glycerol, saline, ethanol, and combinations thereof. The carrier can contain additional agents such as wetting or emulsifying agents, pH buffering agents, or adjuvants which enhance the effectiveness of the formulation. Adjuvants could for example be selected from the group consisting of: AlK(SO4)2, AlNa(SO4)2, AlNH4 (SO4), silica, alum, Al(OH)3, Ca3(PO4)2, kaolin, carbon, aluminum hydroxide, muramyl dipeptides, N-acetyl-muramyl-L-threonyl-D-isoglutamine (thr-DMP), N-acetyl-nornuramyl-L-alanyl-D-isoglutamine (CGP 11687, also referred to as nor-MDP), N-acetylmuramyul-L-alanyl-D-isoglutaminyl-L-alanine-2-(1 '2'-dipalmitoyl-sn - glycero-3-hydroxphosphoryloxy)-ethylamine (CGP 19835A, also referred to as MTP-PE), RIBI (MPL+TDM+CWS) in a 2 percent squalene/Tween-80^{®}emulsion, lipopolysaccharides and its various derivatives, including lipid A, Freund's Complete Adjuvant (FCA), Freund's Incomplete Adjuvants, Merck Adjuvant 65, polynucleotides (for example, poly IC and poly AU acids), wax D from Mycobacterium, tuberculosis, substances found in *Corynebacterium parvum, Bordetella pertussis,* and members of the genus Brucella, Titermax, ISCOMS, Quil A, ALUN, Lipid A derivatives, choleratoxin derivatives, HSP derivatives, LPS derivatives, synthetic peptide matrixes or GMDP, interleukin 1, interleukin 2, Montanide ISA-51 and QS-21, CpG oligonucleotide, poly I:C and GM-CSF. *See* Hunter R, US 5,554,372, and Jager E, Knuth A, WO1997028816.

In a preferred embodiment the immunogenic composition or the vaccine composition of the invention comprises an immunogenically amount of virus like-particles of the invention and is devoid of adjuvant, in a more preferred embodiment,is devoid of aluminum phosphate.

The immunogenic composition or vaccine composition can be administered by any means known to one skilled in the art, such as by intramuscular, subcutaneous or intravenous injection, and oral, nasal, or anal administration. *See* Banga A, "Parenteral Controlled Delivery of Therapeutic Peptides and Proteins," in Therapeutic Peptides and Proteins (Technomic Publishing Co., Inc., Lancaster, PA, US, 1995). To extend the time during which the peptide or protein is available to stimulate a response, the peptide or protein can be provided as an implant, an oily injection, or as a particulate system. The particulate system can be a microparticle, a microcapsule, a microsphere, a nanocapsule, or similar particle. *See* Banga, 1995, *supra.* A particulate carrier based on a synthetic polymer has been shown to act as an adjuvant to enhance the immune response, in addition to providing a controlled release. Aluminum salts can also be used as adjuvants to produce an immune response.

Immunogenic compositions or vaccine compositions can be formulated in unit dosage form, suitable for individual administration of precise dosages. In pulse doses, a bolus administration of an immunogenic composition that includes a disclosed immunogen is provided, followed by a time-period wherein no disclosed immunogen is administered to the subject, followed by a second bolus administration. A therapeutically effective amount of an immunogenic composition can be administered in a single dose, or in multiple doses, for example daily, during a course of treatment. In specific, non-limiting examples, pulse doses of an immunogenic composition that include a disclosed immunogen are administered during the course of a day, during the course of a week, or during the course of a month.

Immunogenic compositions can be administered whenever the effect (such as decreased signs, symptom, or laboratory results of HIV-1 infection, as a non-limitative example) is desired. Generally, the dose is sufficient to treat or ameliorate symptoms or signs of disease without producing unacceptable toxicity to the subject. Systemic or local administration can be utilized.

Amounts effective for therapeutic use can depend on the severity of the disease and the age, weight, general state of the patient, and other clinical factors. Thus, the final determination of the appropriate treatment regimen will be made by the attending clinician. Typically, dosages used *in vitro* can provide useful guidance in the amounts useful for in situ administration of the pharmaceutical composition, and animal models may be used to determine effective dosages for treatment of particular disorders. *See* Gilman R, et al., Eds., Goodman and Gilman's: The Pharmacological Basis of Therapeutics, 8th Ed. (Pergamon Press, New York, NY, US, 1990), and Gennaro A, Ed., Remington's Pharmaceutical Sciences, 18th Ed. (Mack Publishing Co., Easton, PA, US, 1990). Typically, the dose range is from about 0.1 µg/kg body weight to about 100 mg/kg body weight. Other suitable ranges include doses of from about 1 µg/kg to 10 mg/kg body weight. In one example, the dose is about 1.0 µg to about 50 mg, for example, 1 µg to 1 mg, such as 1 mg peptide per subject. The dosing schedule can vary from daily to as seldom as once a year, depending on clinical factors, such as the subject's sensitivity to the peptide and tempo of their disease. Therefore, a subject can receive a first dose of a disclosed therapeutic molecule, and then receive a second dose (or even more doses) at some later time(s), such as at least one day later, such as at least one week later.

All the terms and embodiments previously described are equally applicable to the immunogenic composition or the vaccine composition of the invention.

### Therapeutic methods of the invention

In another aspect, the invention relates to fusion protein as defined in the invention, a polynucleotide as defined in the invention, a vector as defined in the invention, a host cell as defined in the invention, a virus like-particle as defined in the invention or an immunogenic composition or vaccine composition as defined in the invention for use in medicine

In another aspect, the invention relates to a fusion protein as defined in the invention, a polynucleotide as defined in the invention, a vector as defined in the invention, a host cell as defined in the invention, a virus like-particle as defined in the invention or an immunogenic composition or vaccine composition as defined in the invention for use the prevention and/or treatment of a disease caused by an infection in a subject, wherein the infection is by a pathogen which contains the immunogenic polypeptide forming part of the fusion protein or a region thereof.

Alternatively, the invention relates to a method for prevention and/or treatment of a disease caused by an infection in a subject, wherein the infection is by a pathogen which contains the immunogenic polypeptide forming part of the fusion protein or a region thereof comprising administering to the subject a therapeutically effective amount of fusion protein as defined in the invention, a polynucleotide as defined in the invention, a vector as defined in the invention, a host cell as defined in the invention, a virus like-particle as defined in the invention or an immunogenic composition or vaccine composition as defined in the invention.

Alternatively, the invention relates to the use of a fusion protein as defined in the invention, a polynucleotide as defined in the invention, a vector as defined in in the invention, a host cell as defined in the invention, a virus like-particle as defined in the invention or an immunogenic composition or vaccine composition as defined in the invention for the preparation of a medicament for preventing and/or treating of a disease caused by an infection in a subject, wherein the infection is by a pathogen which contains the immunogenic polypeptide forming part of the fusion protein or a region thereof.

In a preferred embodiment, the immunogenic polypeptide forming part of the fusion protein is an HIV polypeptide and the infection is an HIV infection. In a more preferred embodiment, the HIV polypeptide comprises the membrane proximal external region of gp41 or an immunologically equivalent variant thereof.

In a preferred embodiment the subject is human, chimpanzee, macaque or baboon. In a more preferred embodiment of the invention, the subject is a human.

Infectious diseases may be caused by viruses including adenovirus, cytomegalovirus, dengue, Epstein-Barr, hanta, hepatitis A, hepatitis B, hepatitis C, herpes simplex type I, herpes simplex type II, human immunodeficiency virus (HIV), human papilloma virus (HPV), influenza, measles, mumps, papova virus, polio, respiratory syncytial virus, rinderpest, rhinovirus, rotavirus, rubella, SARS virus, smallpox, viral meningitis, and the like. Infectious diseases may also be caused by bacteria including *Bacillus antracis, Borrelia burgdorferi, Campylobacter jejuni*, *Chlamydia trachomatis*, *Clostridium botulinum, Clostridium tetani, Diptheria, E. coli, Legionella, Helicobacter pylori, Mycobacterium rickettsia, Mycoplasma nesisseria, Pertussis*, *Pseudomonas aeruginosa, S. pneumonia*, *Streptococcus, Staphylococcus*, *Vibria cholerae, Yersinia pestis,* and the like. Infectious diseases may also be caused by fungi such as *Aspergillus fumigatus*, *Blastomyces dermatitidis, Candida albicans, Coccidioides immitis, Cryptococcus neoformans, Histoplasma capsulatum*, *Penicillium marneffei,* and the like. Infectious diseases may also be caused by protozoa and parasites such as *chlamydia*, *kokzidioa, leishmania, malaria, rickettsia, trypanosoma,* and the like.

The present invention further relates to preventing or reducing symptoms associated with HIV infection. These include symptoms associated with the minor symptomatic phase of HIV infection, including, for instance, shingles, skin rash and nail infection, mouth sores, recurrent nose and throat infection, and weight loss. In addition, further symptoms associated with the major symptomatic phase of HIV infection, include, for example, oral and vaginal thrush (Candida), persistent diarrhea, weight loss, persistent cough, reactivated tuberculosis, and recurrent herpes infections, such as cold sores (herpes simplex). Symptoms of full-blown AIDS which can be treated in accordance with the present invention, include, for instance, diarrhea, nausea and vomiting, thrush and mouth sores, persistent, recurrent vaginal infections and cervical cancer, persistent generalized lymphadenopathy (PGL), severe skin infections, warts and ringworm, respiratory infections, pneumonia, especially *Pneumocystis carinii pneumonia* (PCP), herpes zoster (or shingles), nervous system problems, such as pains, numbness or "pins and needles" in the hands and feet, neurological abnormalities, Kaposi's sarcoma, lymphoma, tuberculosis, and other opportunistic infections.

The fusion protein as defined in the invention, the polynucleotide as defined in the invention, the vector as defined in the invention, the host cell as defined in the invention, the virus like-particle as defined in the invention or the immunogenic composition or vaccine composition of the invention are capable of inducing neutralizing antibodies in the subject.

Typically, the neutralizing antibodies used in the method of the present invention bind to the surface of the pathogen and inhibit or reduce infection by the pathogen by at least 99 percent, at least 95 percent, at least 90 percent, at least 85 percent, at least 80 percent, at least 75 percent, at least 70 percent, at least 60 percent, at least 50 percent, at least 45 percent, at least 40 percent, at least 35 percent, at least 30 percent, at least 25 percent, at least 20 percent, or at least 10 percent relative to infection by the pathogen in the absence of said antibody(ies) or in the presence of a negative control. The nAbs can then be tested to determine if they have a neutralizing activity or bNAb activity using any of the methods provided herein. If the neutralizing antibodies or BNAbs were raised in a non-human animal, the CDRs or the complete sequence can be transferred from the non-human framework to a human framework to generate an antibody suitable for administration to a human. Methods for determining whether an antibody is a nAb have been described in the art. *See* Li M, et al., J. Virol. 2005; 79:10108-10125, Wei X, et al., Nature 2003; 422:307-312, and Montefiori D, Curr. Protoc. Immunol. 2005; Jan, Chapter 12:Unit 12.11. These methods are based on the determination of the reduction in expression of a reporter gene after a single round of viral infection using a receptive cell line using a virus which encodes the reporter gene.

In another preferred embodiment the fusion protein, the polynucleotide, the vector, the host cell or the virus like-particle is for use according to the invention, wherein the subject is previously treated with a conjugate comprising the immunogenic polypeptide forming part of the fusion protein coupled to a carrier.

In a preferred embodiment, the immunogenic polypeptide corresponds to any region of HIV gp41 polypeptide or a functionally equivalent variant thereof. In a more preferred embodiment, the region is the MPER sequence shown in SEQ ID NO : 23

In another preferred embodiment, the carrier is KLH (Keyhole Limpet hemocyanin)

In another preferred embodiment of the medical uses of the invention, the virus-like particle is administered or is to be administered in the absence of aluminium phosphate.

The invention is illustrated by way of the following examples which are to be construed as merely illustrative and not limitative of the scope of the invention.

### EXAMPLES

### Materials and methods

### Expression vectors for small gp41-derived proteins, gag and fusion proteins

The pMIN mammalian expression plasmid coding for a small protein containing the C-terminal Heptad Repeat (HR2), the Membrane Proximal External Region (MPER), the Transmembrane Domain (TM) has been described previously (Figure 1) [Molinos-Albert LM, et al. Retrovirology 2014, 11:44]. A similar plasmid pMINFULL coding for a gp41-derived protein that contains the MIN sequence and the cytoplasmic tail of gp41 was also generated (Figure 1). MIN sequence was cloned in a pET-21D+expression vector (Novagen) for *E. coli* production of recombinant proteins.

Gag sequences were amplified from the pGag-EGFP plasmid that codes for a Rev-independent HIV-1 Gag protein fused in frame to the enhanced GFP. The plasmid was obtained from the NIH AIDS Reagent Program (Cat #11468) [Dhillon AK, et al. J Virol 2007, 81:6548-6562]. The inventors generated a pGAG plasmid coding for the full Gag sequence and a plasmid coding for a fusion protein MINGAG (Figure 1). Briefly, the HR2-TM fragment of Gp41 was amplified from the pMIN plasmid and the HIV-1 gag gene (starting at residue 2) was amplified from the pGag-EGFP plasmid. MIN and Gag amplicons were fused by PCR and the final product was cloned into a pcDNA 3.1 using Directional TOPO® Expression Kit (Invitrogen, Carlsbad, CA, USA; Cat. Nos. K4900-01, K4900-40) to generate the pcDNA3.1/MINGAG-3.7 plasmid (Figure 1). All amplification reactions were performed using Amplitaq DNA polymerase (Applied Biosystems, Foster City, CA, USA) in a 2720 Thermal cycler (Applied Biosystems, Foster City, CA, USA). All plasmids were sequenced to confirm the expected sequence and insertion sites.

### Peptides, gp41-derived proteins and of VLP

The C34, T-20 and MPER peptides (HXB2 sequence) and Keyhole Limpet hemocyanin (KLH) and MPER coupled to KLH (KLH-MPER) were from Thermo Fisher (Germany). 15-mer overlapping peptides covering the gp41 HR2 and MPER regions were kindly provided by Christian Brander (IrsiCaixa, Badalona, Spain). The Min protein containing a C-terminal 6xHis tag (Figure 1A) was produced in *E. coli* BL21 DE3 strain (Invitrogen). Inclusion bodies were obtained from a 1 L culture bacterial extract and solubilised by using an 8M urea solution. Highly pure protein was obtained through niquel-based Immobilized Metal Affinity Chromatography (GE Healthcare) and gel filtration using a Sephacryl S-200 HR column (GE Healthcare).

VLP were obtained by transfection of different derivatives of HEK293 cells according to the production scale. For stable transfection, we transfected 293T cells with pMINFULL and pGAG, pGAG or pcDNA3.1/MINGAG-3.7 using calphos mammalian transfection kit (Clontech), and selected the transfected cells by using geneticine plus hygromicine, hygromicine or geneticine, respectivetly.

Given the poor yield of MINGAG VLPs, Large scale production of GAG and MINGAG VLPs was carried out in a different setting. Large amounts of the pcDNA3.1/MINGAG-3.7 expression plasmid were produced using maxiprep kits (QIAGEN) and were used for transient transfection of HEK-293T cells. Gag VLP were produced in 1 L culture flask, while MINGAG VLP were produced in a 5 L bag. 48 hours after transfection, supernatants were collected, ultrafiltered, concentrated (to 1 L in the case of MINGAG) and ultracentrifuged. Both preparations contained large amounts of gag protein as assessed by Nanoparticle Tracking Analysis (NTA) by Nanosight® and electron microscopy.

### Immunogenicity studies

Female C57BL/6 mice (Harlan) aged 6-8 weeks were used for murine immunization protocols. Groups of mice (5 mice per group) were subdermally immunized with 50 µg of recombinant proteins or VLPs at 3-week intervals. Blood samples were collected by maxillary artery puncture before immunization and at 3 weeks. Mice were observed daily to record body weight changes. All animal experiments and husbandry involved in the studies were conducted under the Catalan Government's guidelines for the use and care of laboratory animals and all procedures were approved by the Germans Trias I Pujol Research Institute (IGTP) Animal Care and Use Committee.

For rabbit immunization, 3- to 4-month-old New Zealand White rabbits were inoculated subcutaneously with different immunogens at the animal facility of the University of Barcelona. A control rabbit group (n=4) was primed with full gag DNA and Nude VLP, and the immunized twice at weeks 0 and 3 with Nude VLP. A second group of rabbits (n=4) was primed with DNA coding for HTI immunogen [Mothe B. et al., J Transl Med. 2011 Dec 7;9:208.], full HIV *gag* and full HIV *env* genes and then immunized at week 0 and 3 with MINGAG VLP. Sera were collected at weeks 1, 3, 4 and at sacrifice. All animal experiments and husbandry involved in the studies were conducted under the Catalan Government's guidelines for the use and care of laboratory animals and all procedures were approved by the University Animal Care and Use Committee.

**Enzyme Linked Immunosorbent Assays.** Peptides C34, T20, MPER, overlapping 15mer gp41 peptides or recombinant GAG or MIN protein were used to coat 96-well Maxisorp Nunc-immuno plates (Fisher Scientific). After blocking, plates were incubated with 100ul of previously diluted plasma samples overnight at 4°C. Plates were then washed and 100ul of a Horseradish Peroxidase (HRP)-conjugated F(ab)2 Goat antimouse IgG (Fc specific, Jackson Immunoresearch) were dispensed for one hour at room temperature. Plates were developed with 100 µl of O-Phenylenediamine dihydrochloride (OPD) substrate (Sigma-Aldrich) and stopped with 100 µl of 4N H₂SO₄. Optical density was measured at 492 nm for specific signal and at 620nm for background.

**Viruses and neutralization assays.** HIV-2 chimeras were made in the context of the full-length p7312A HIV-2 molecular clone (GenBank accession number L36874). Expression vectors for the wild type HIV-2 (p7312A) and HIV-2 chimeras containing the HIV-1 gp41 Membrane Proximal External Region (p7312A-C1), the 2F5 (p7312A-C3) or 4E10 epitopes (p7312A-C4), were kindly provided by G.M Shawn (University of Pennsylvania) [Dhillon AK, et al. cited supra]. Pseudoviruses were generated by transfection of plasmids in 293T cells. After 24 hours post-transfection, supernatants were harvested, filtered at 0.45 micron and viral stocks frozen at -80°C.

HIV-1 isolates NL4.3, BaL, AC10 and SVPB16 were generated as pseudoviruses using Env expression plasmids and the pSG3 vector as described [Sánchez-Palomino S, et al. Vaccine 2011, 29:5250-5259]. Cell-free virus neutralization by plasma samples was tested by a standard TZM-bl based assay [Sánchez-Palomino S, et al. cited supra]. Briefly, in a 96-well culture plate, 100ul of previously diluted plasma samples were preincubated with 50ul of pseudovirus stock, using 200 TCID50, at 37°C, one hour. Then, 100 µl containing 10,000 TZM-bl luciferase-reporter target cells per well were added. Plates were cultured at 37°C and 5% CO2 for 48 hours. 2F5, 4E10 and IgGb12 (Polymun Scientific), and anti-CD4 clone SK3 (BD Biociences) were used as controls. Plasma samples were inactivated (56°C, 30 minutes) prior to the assay. TZM-bl reporter cells were treated with dextran (Sigma Aldrich) to enhance infectivity. Luciferase substrate, Britelite Plus (Perkin-Elmer) was used for the read out of HIV infectivity.

### Example 1- Generation of VLP presenting HIV MPER epitopes

The inventors recently reported the characterization of several gp41-derived miniproteins containing the neutralizing epitope MPER. Among them, the protein MIN was selected according to its higher expression and proper MPER exposure on the cell-surface of transfected cells [Molinos-Albert LM, et al cited supra]. As a first approach to test its potential immunogenicity, the inventors produced both recombinant MIN protein and VLP displaying the selected MIN protein (Figure 1). For recombinant MIN protein, production was carried out in *E. coli* and protein was purified to homogeneity, showing the expected 17 kDa molecular weight (Figure 2A). The immunogenicity of recombinant MIN protein was assayed in C57BL/6 mice. Animals immunized with recombinant preparation of MIN (3 doses, 20 µg each) showed IgG reactive against this protein that were increased by the presence of aluminum phosphate as adjuvant (Figure 2B).

For VLPs, the cytoplasmic domain of gp41 was added to the MIN sequence (MINFULL construction) in order to increase interaction with Gag during VLP budding. A 293T cell clone stably expressing both MINFULL and GAG HIV-1 proteins was selected according to protein expression and VLP production. The selected clone (4G2A) showed cell surface MPER exposure and an intracellular GAG content similar to a GAG expressing 293T cell clone selected in parallel and lacking gp41 expression (Figure 2B). Supernatants from these clones contained gag protein mostly in a high molecular weight form (>300 kDa) suggesting the proper formation of VLP (Data not shown). This observation was further confirmed by the WB analysis of centrifuged VLP and cellular lysates that contained GAG protein in control cells and both GAG and GP41 proteins in the MINFULL and GAG coexpressing cells (Figure 2C). In immunogenicity assays, C57BL/6 mice immunized with MIN-VLPs (three doses 10 µg of total protein each) in the presence or absence of adjuvant (aluminum phosphate) showed non-significant anti gp41 IgG response compared to control animals (Figure 2E), despite the fact that a high reactivity against 293T cell surface proteins and HIV-1 GAG antigen could be readily detected (data not shown). Furthermore, no significant anti-HIV-1 neutralizing activity was detected in any animal, either immunized with MIN recombinant protein or VLP, when assayed against a mini panel of four viruses (NL4-3, BaL, AC-10 and SVBP16; data not shown).

### Example 2 Engineered VLPs: self-packing gp41-gag fused proteins

Considering the low amounts of MIN protein incorporated in VLP (Figure 2C) and the low number of Env spikes reported in HIV virions [Zhu P, et al. Nature 2006, 441:847-852], the inventors hypothesized that the poor incorporation of MIN protein into the VLPs could be a limiting factor in the above described approach, and they explored whether increasing the density of MPER epitopes on the surface of VLP could enhance its immunogenic potential. To test such hypothesis, we constructed an expression plasmid (pcDNA3.1/MINGAG-3.7) coding for a fusion protein encompassing the HR2, the MPER and the TM region of gp41 fused to the GAG protein of HIV. The pcDNA3.1/MINGAG-3.7 plasmid, a control pcDNA3.1GAG and an empty plasmid were transfected in 293T cells and the expression of gp41 and Gag epitopes was analyzed either by flow cytometry or western-blot. Figure 3A shows the specific cell surface staining using two anti MPER antibodies (10E8 and 2F5) of Gag+ cells in 293T cultures transfected with the MINGAG construction. MPER cell surface staining is absent in GAG transfected cells, suggesting that the MINGAG protein is properly expressed and reaches the cell surface. Further confirmation of proper expression was achieved in Western Blot analyses of cell lysates showing the expression of a major protein recognized by anti-gag antibodies with an apparent molecular weight of 65 kDa and 57 kDa in MINGAG and Gag transfected cells respectively. The same lysates showed a 65kDa and a second protein when revealed with the 2F5 antibody (Figure 3B).

The release of VLP by transfected cells was assessed by ELISA in cleared supernatant collected 48 hours after transfection. Although VLP production was 8-fold lower for the MINGAG construct than for Nude VLPs (Figure 3C), both proteins showed similar levels of particulate material, suggesting proper particle generation. Further characterization of particle diameter by Nanosight showed similar mean diameter: 160 +/- 40 nm for Nude VLPs and 170 +/- 50 nm for MINGAG VLPs (Mean +/- SD of four experiments, Figure 3D).

### Example 3- Immunogenicity of VLP-Gag and VLP-MinGag in mice.

The inventors tested immunogenicity of MINGAG VLPs in C57BL/6 mice. Five animals per group were injected with a 200 µl preparation of Nude or MINGAG VLPs with or without aluminum phosphate as adjuvant. Immunogens were prepared in order to maintain the same Gag antigen concentration between groups, whereby the only difference was the presence or not of the MIN fragment in MINGAG VLPs. According with this, all animals were injected with four doses (at 3 weeks interval) of 32ng of gag antigen.

The time course of anti gp41 IgG responses (measured using the MIN antigen) showed high and specific immunogenicity in both MINGAG immunized groups, with a faster elicitation in adjuvanted animals but higher final levels in animals immunized only with VLP (Figure 4A). Conversely, anti p24-Gag IgG that were similar in animals immunized with Nude or MINGAG-VLPs and were higher in both groups of adjuvanted animals (Figure 4B). We tested whether the enhancing effect of adjuvant on anti Gag responses and the inhibitory effect on anti gp41 responses could be due to undesired effects on VLP structure. Electron microscopy analysis showed that aluminum phosphate treated VLP lost native structure suggesting that release of GAG content could explain these results (Figure 4C).

These results indicate that the preparation of MINGAG VLPs is immunogenic, as some of the animals immunized with this preparation were able to mount a strong IgG response. However, no significant neutralizing activity was observed in both sera and purified IgG obtained from control or immunized animals against two laboratory adapted viruses (NL43 and BaL, Figure 4D).

To increase the response to the MPER of our MINGAG protein, the inventors designed a second immunization procedure in which animals were immunized with MPER peptides coupled to KLH (four doses every three weeks) before VLP immunization (similar to the above described immunization) in the absence of adjuvant. Again, all animals showed similar anti Gag responses (data not shown), while only MINGAG immunized animals showed strong anti gp41 IgG responses (Figure 5A). Mapping of anti gp41 IgG responses showed two main immunogenic areas, one of them covering the HR2 region, and the second one in the MPER sequence overlapping with the 2F5 epitope (Figure 5B). Consistently, a significant neutralizing activity was observed in sera from MINGAG VLP immunized animals compared to control mice (Figure 5C). Neutralization was observed against the BaL, AC10 and SVBP16 isolates of HIV (all subtype B) and was specifically elicited by immunization since it was retained in the IgG fraction of sera and was absent in preimmune sera (Data not shown).

### Example 4- Immunogenicity of VLP-Gag and VLP-MinGag in rabbits.

A similar approach was used to test immunogenicity of MINGAG VLP in rabbits. VLP were injected (two doses of MINGAG VLPs) in the absence of adjuvants and animals were primed with gag and env DNA. All animals generated significant anti gp41 IgG responses (as measured by the T-20 peptide and the C34 peptides, Figure 6A) that mapped to the HR2 and the MPER sequences of the immunogen protein, with some overlap with the 2F5 neutralizing epitope (Figure 6B). Again, a significant neutralizing activity was observed in sera from MINGAG VLP immunized animals compared to control rabbits (Data not shown). Neutralization was observed against the NL4-3 isolate, was retained in the IgG fraction, and was specific since no significant activity was detected against VSV pseudotyped viruses (Figure 6C).

## Claims

1. A fusion protein comprising a HIV gag polypeptide or a functionally equivalent variant thereof and an immunogenic polypeptide, wherein the immunogenic polypeptide is located N-terminal to the HIV gag polypeptide or to the functionally equivalent variant thereof.

2. The fusion protein according to claim 1 wherein the immunogenic polypeptide does not contain an epitope from the HIV gag polypeptide.

3. The fusion protein according to claims 1 or 2 wherein the fusion protein contains a transmembrane domain between the HIV gag polypeptide or a functionally equivalent variant and the immunogenic polypeptide.

4. The fusion protein according to claim 3 wherein the functionally equivalent variant of HIV gag lacks the myristoylation sequence.

5. The fusion protein according to claims 1 to 4 further comprising a linker, preferably the linker comprises glycine residues, more preferably, the linker comprises glycine residues and serine residues, even more preferably, the linker comprises three glycine residues and one serine residue, even more preferably, the linker comprises the sequence shown in SEQ ID NO: 24 or the sequence shown in SEQ ID NO: 25.

6. The fusion protein according to claims 3 to 5 wherein the transmembrane domain is the transmembrane domain of the HIV gp41 polypeptide or a functionally equivalent variant thereof

7. The fusion protein according to any of claims 1 to 6 wherein the immunogenic polypeptide is from a protein of a virus and/or the functionally equivalent variant of HIV gag has the sequence shown in SEQ ID NO: 2.

8. The fusion protein according to claim 7 wherein the virus is HIV.

9. The fusion protein according to claim 8 wherein the protein of the HIV virus is env or a product results from the processing of the env protein, preferably the env protein is gp41.

10. The fusion protein according to claim 9 wherein the immunogenic polypeptide comprises from the N-to the C-terminus
a) the heptad repeat 2 of the HIV gp41 polypeptide or an immunologically equivalent variant thereof, and
b) the membrane proximal external region of the HIV gp41 polypeptide or an immunologically equivalent variant thereof.

11. The fusion protein according to claim 10 of sequence SEQ ID NO: 29.

12. A polynucleotide encoding a fusion protein according to any of claims 1 to 11.

13. The polynucleotide according to claim 12 of sequence SEQ ID NO: 9.

14. The polynucleotide according to claim 13 whose sequence is codon-optimised for expression in humans.

15. A vector comprising a polynucleotide according to any of claims 12 to 14.

16. A host cell comprising a fusion protein according to any of claim 1 to 11, a polynucleotide according to any of claims 12 to 14 or a vector according to claim 15.

17. A method for preparing a VLP loaded with an immunogenic polypeptide comprising
(i) expressing in a cell a fusion protein according to any of claims 1 to 11 and
(ii) recovering the VLPs from the extracellular medium.

18. A virus like-particle comprising the fusion protein according to any of claims 1 to 11 or obtained using a method as defined in claim 17.

19. An immunogenic composition or a vaccine composition comprising an immunogenically amount of a fusion protein as defined in any of claim 1 to 11, a polynucleotide as defined in any of claims 12 to 14, a vector as defined in claim 15, a host cell as defined in claim 16 or a virus like-particle according to claim 18.

20. An immunogenic composition or a vaccine composition comprising an immunogenically amount of virus like-particles according to claim 18 and is devoid of adjuvant.

21. An immunogenic composition or a vaccine composition according to claim 20, wherein the adjuvant is aluminum phosphate.

22. A fusion protein as defined in any of claims 1 to 11, a polynucleotide as defined in any of claims 12 to 14, a vector as defined in claim 15, a host cell as defined in claim 16, a virus like-particle as defined in claim 18 or the immunogenic composition or a vaccine composition as defined in any of claims 19-21 for use in medicine.

23. A fusion protein as defined in any claims 1 to 11, a polynucleotide as defined in any of claims 12 to 14, a vector as defined in claim 15, a host cell as defined in claim 16, a virus like-particle according to claim 18 or the immunogenic composition or a vaccine composition as defined in any of claims 19-21 for use in the prevention and/or treatment of a disease caused by an infection in a subject, wherein the infection is by a pathogen which contains the immunogenic polypeptide forming part of the fusion protein or a region thereof preferably the immunogenic polypeptide forming part of the fusion protein is a HIV polypeptide and the infection is a HIV infection, more preferably the HIV polypeptide comprises the membrane proximal external region of gp41 or an immunologically equivalent variant thereof.

24. A fusion protein, a polynucleotide, a vector, a host cell, a virus like-particle or the immunogenic composition or a vaccine composition for use according to any of claims 22 to 23, wherein the subject is previously treated with a conjugate comprising the immunogenic polypeptide forming part of the fusion protein coupled to a carrier, preferably the carrier is KLH.

25. A virus like-particle for use according to any of claims 22 to 24, wherein said virus like-particle is administered in the absence of aluminum phosphate.
